# EUROPÄISCHE PATENTANMELDUNG

(11) **EP 1 847 182 A2**
(43) Veröffentlichungstag der Anmeldung: **24.10.2007**
(21) Anmeldenummer: 07015474.5
(22) Anmeldetag: 06.02.2002
(51) Int. Cl.: A23L 1/30

(54) **Nahrungsmittel oder Nahrungsergänzungsmittel enthaltend Flavonoid-Derivate**

(30) Priorität: 02.03.2001 DE 10110105
(62) Teilanmeldung aus: 02710842.2
(71) Anmelder: Merck Patent GmbH, 64293 Darmstadt (DE)
(72) Erfinder: Wirth, Corinna, Dr., 69115 Heidelberg (DE); Buchholz, Herwig, Dr., 60599 Frankfurt (DE); Carola, Christophe, Dr., 69120 Heidelberg (DE)

(57) **Zusammenfassung**

Es werden Nahrungsmittel oder Nahrungsergänzungsmittel beschrieben, die mit einem oder mehreren Flavonoid-Derivaten angereichert sind oder diese enthalten.

## Beschreibung

Die Erfindung betrifft Nahrungsmittel oder Nahrungsergänzungsmittel enthaltend Flavonoid-Derivate bzw. die Verwendung der Flavonoid-Derivate in Nahrungsmitteln oder Nahrungsergänzungsmitteln.

Eine mehr oder minder stark ausgeprägte Sonnenbräune der Haut gilt in der modernen Gesellschaft als attraktiv und als Ausdruck von Dynamik und Sportlichkeit. Neben dieser erwünschten Wirkung der Sonne auf die Haut treten eine Reihe von unerwünschten Nebenwirkungen auf, wie Sonnenbrand oder vorzeitige Hautalterung und Faltenbildung. lnzwischen sind eine Anzahl von leistungsfähigen UV-Filtern entwickelt worden, die in Form von Cremes, Lotionen oder Gelen auf die Haut aufgetragen auch bei stärkerer Sonneneinwirkung die Entwicklung von Sonnenbrand wirksam verzögern können. Der in der pharmazeutischen oder kosmetischen Zubereitung enthaltene UV-Filter bildet auf der Oberfläche der Haut einen Film bzw. eine Schicht aus und dringt nicht mit weiteren in der Zubereitung enthaltenen pflegenden Substanzen in tiefere Hautschichten vor. Bekannte UV-Filter bzw. Sonnenschutzmittel wirken also in der Weise, dass sie bestimmte Bereiche des Sonnenlichts absorbieren und somit diese Strahlung nicht in tiefere Schichten der Haut vordringen kann. Bekanntlich wird der gefährlichste Teil der Sonnenstrahlung von den ultravioletten Strahlen mit einer Wellenlänge von weniger als 400 nm gebildet. Die untere Grenze der ultravioletten Strahlen, welche die Erdoberfläche erreichen, ist durch die Absorption in der Ozonschicht auf ca. 280 nm beschränkt. Die heute in der Kosmetik üblichen Sonnenschutzfilter absorbieren in einem Wellenlängenbereich von 280 bis 400 nm. Dieser Bereich umfasst UV-B-Strahlen mit einer Wellenlänge zwischen 280 und 320 nm, die bei der Bildung eines Sonnenerythems eine entscheidende Rolle spielen, wie auch UV-A-Strahlen, mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische und irritative Reaktionen auslösen können.

Hautschädigungen werden nicht nur durch Sonnenlicht verursacht, sondern auch durch andere äußere Einflüsse, wie Kälte oder Wärme. Ferner unterliegt die Haut einer natürlichen Alterung, wodurch Falten entstehen und die Spannkraft der Haut nachlässt.

Aufgabe pflegender Kosmetik ist es, nach Möglichkeit den Eindruck einer jugendlichen Haut zu erhalten. Prinzipiell stehen verschiedene Wege offen, um dieses Ziel zu erreichen. So können bereits vorhandene Schädigungen der Haut, wie unregelmäßige Pigmentierung oder Faltenbildung, durch abdeckende Puder oder Cremes ausgeglichen werden. Ein anderer Ansatzpunkt ist, die Haut vor Umwelteinflüssen zu schützen, die zu einer dauerhaften Schädigung und damit Alterung der Haut führen. Die Idee ist also, vorbeugend einzugreifen und dadurch den Alterungsprozess hinauszuzögern. Ein Beispiel hierfür sind die bereits erwähnten UV-Filter, welche durch Absorption bestimmter Wellenlängenbereiche eine Schädigung der Haut vermeiden oder zumindest vermindern. Entsprechend der Lage ihrer Absorptionsmaxima werden UV-Absorber für kosmetische und dermatologische Zubereitungen in UV-A- und UV-B-Absorber eingeteilt, wobei UV-A-Absorber üblicherweise auch im UV-B-Bereich absorbieren und daher alternativ auch als Breitbandabsorber oder -filter bezeichnet werden.

Die bekannten UV-Filter haben jedoch oftmals Nachteile: beispielsweise ist ihre Hautverträglichkeit nicht befriedigend oder ihre Absorptionseigenschaften sind ungenügend. Die ungenügenden Absorptionseigenschaften können sich z.B. darin äußern, dass nur ein geringer Teil des UV-Spektrums absorbiert wird oder dass der Absorptionskoeffizient bei einer gegebenen Wellenlänge nicht befriedigend ist.

Während bei UV-Filtern das schädigende Ereignis, die UV-Strahlung, von der Haut abgeschirmt wird, versucht man bei einem weiteren Weg, die natürlichen Abwehr- bzw. Reparaturmechanismen der Haut gegen das schädigende Ereignis zu unterstützen. Schliesslich verfolgt man als weiteren Ansatzpunkt die mit zunehmendem Alter sich abschwächenden Abwehrfunktionen der Haut gegen schädigende Einflüsse auszugleichen, indem Substanzen von aussen zugeführt werden, die diese nachlassende Abwehr- bzw. Reparaturfunktion ersetzen können. Beispielsweise besitzt die Haut die Fähigkeit, Radikale, die durch äussere oder innere Stressfaktoren erzeugt werden, abzufangen. Diese Fähigkeit schwächt sich mit zunehmendem Alter ab, wodurch sich der Alterungsprozess mit zunehmendem Alter beschleunigt.

Eine weitere Schwierigkeit bei der Herstellung von Kosmetika besteht darin, dass Wirkstoffe, die in kosmetische Formulierungen eingearbeitet werden sollen, oftmals nicht stabil sind und in der Formulierung geschädigt werden können. Die Schädigungen können beispielsweise durch eine Reaktion mit Luftsauerstoff oder durch die Absorption von UV-Strahlen verursacht werden. Die so geschädigten Moleküle können durch ihre Strukturänderung z.B. ihre Farbe ändern und/oder ihre Wirksamkeit verlieren.

Aufgabe der vorliegenden Erfindung war es daher, kosmetische Formulierungen zur Verfügung zu stellen, die die Nachteile des Standes der Technik vermeiden und insbesondere vorteilhafte Absorptionseigenschaften besitzen.

Überraschend wurde nun gefunden, dass diese Aufgabe durch die Verwendung der Verbindungen der Formel I 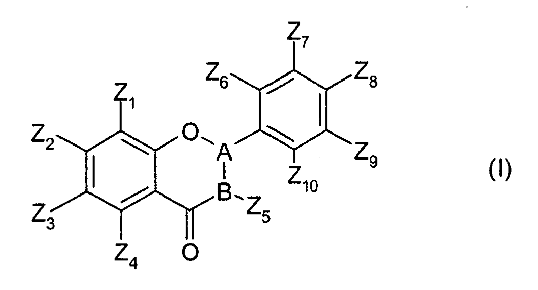 worin
Z₁ bis Z₄ und
Z₆ bis Z₁₀ jeweils unabhängig voneinander H, OH, CH₃COO, Alkoxy, Hydroxyalkoxy, Mono- oder Oligoglykosidreste bedeuten und wobei die Alkoxy- und Hydroxyalkoxygruppen verzweigt und unverzweigt sein und 1 bis 18C-Atome aufweisen können, 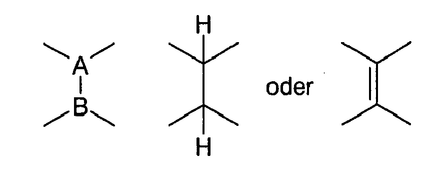 bedeutet,
Z₅ ein Mono- oder Oligoglykosidrest ist, wobei an diesen Glykosidrest jeweils über eine Gruppe -O- mindestens ein Rest gebunden ist, der ausgewählt ist aus 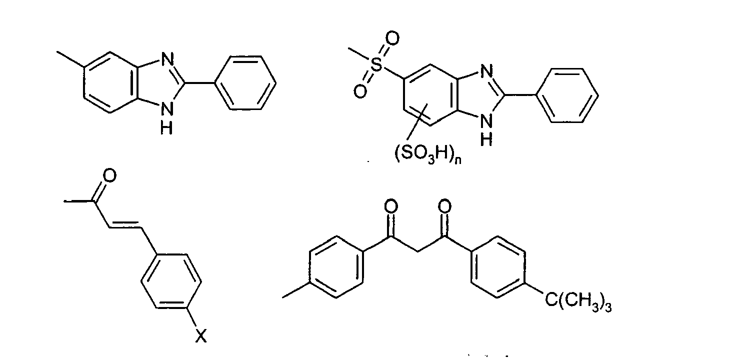 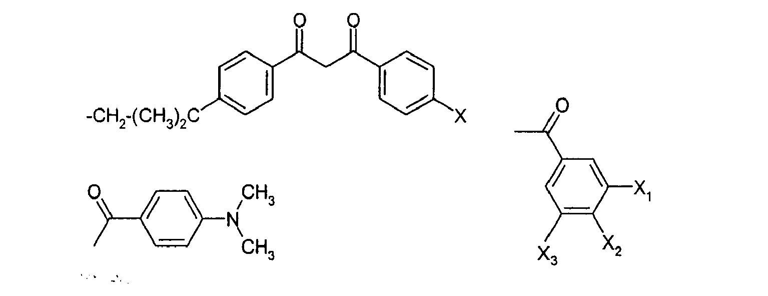 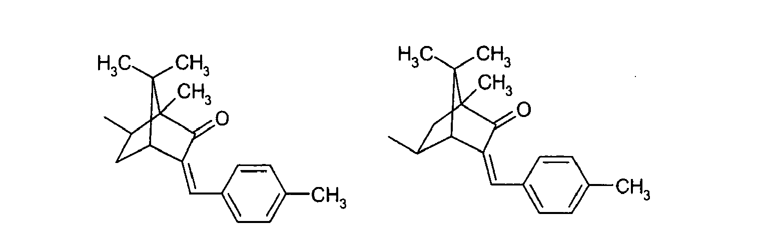 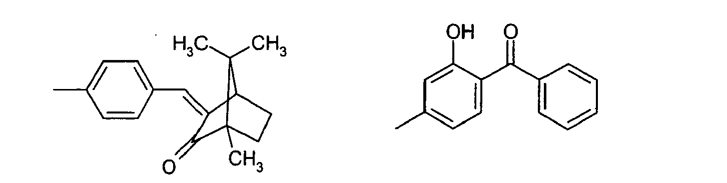 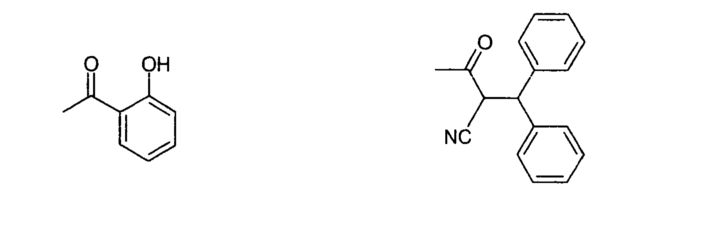 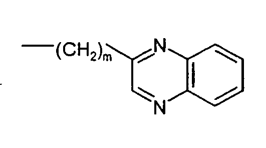 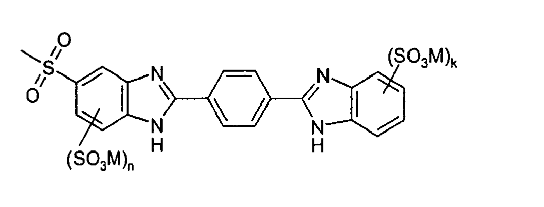 worin X, X₁, X₂ und X₃ jeweils unabhängig voneinander OH, CH₃COO, einen Alkoxyrest mit 1 bis 8 C-Atomen oder einen Monoglykosidrest bedeuten, n 0, 1, 2 oder 3, m 0 oder 1, k 0, 1, 2, 3 oder 4 und M H, Na oder K ist, und
worin ein oder mehrere Wasserstoffatome in den OH-Gruppen der in den Substituenten Z₁ bis Z₁₀ genannten Glykosidreste jeweils unabhängig voneinander auch durch Acetyl oder durch Alkylreste mit 1 bis 8 C-Atomen ersetzt sein können und wobei an ein oder mehrere Hydroxygruppen der in den Substituenten Z₁ bis Z₁₀ genannten Reste jeweils unabhängig voneinander auch Sulfat oder Phosphat gebunden sein kann,
in kosmetischen Formulierungen gelöst wird.

Die Erfindung betrifft somit kosmetische Formulierungen enthaltend eine oder mehrere Verbindungen der Formel I 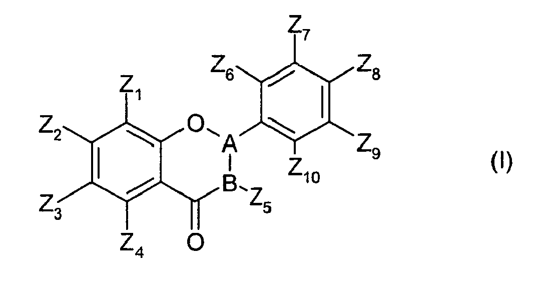 worin
Z₁ bis Z₄ und
Z₆ bis Z₁₀ jeweils unabhängig voneinander H, OH, CH₃COO, Alkoxy, Hydroxyalkoxy, Mono- oder Oligoglykosidreste bedeuten und wobei die Alkoxy- und Hydroxyalkoxygruppen verzweigt und unverzweigt sein und 1 bis 18 C-Atome aufweisen können, 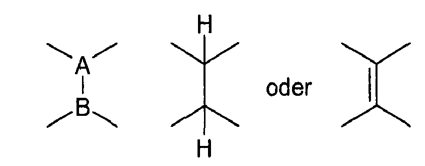 bedeutet,
Z₅ ein Mono- oder Oligoglykosidrest ist, wobei an diesen Glykosidrest jeweils über eine Gruppe -O- mindestens ein Rest gebunden ist, der ausgewählt ist aus 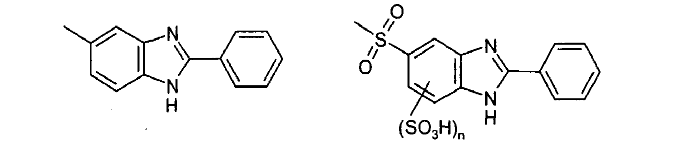 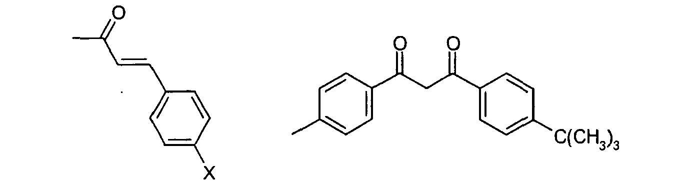 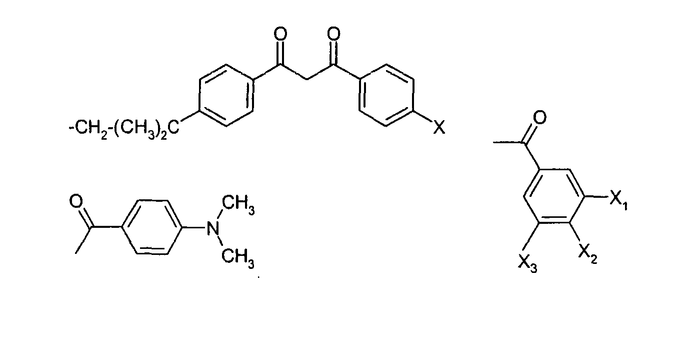 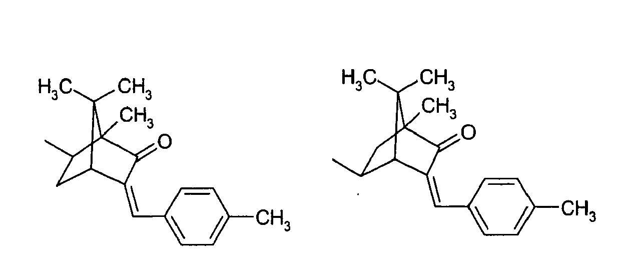 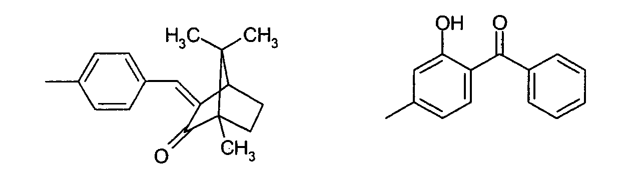 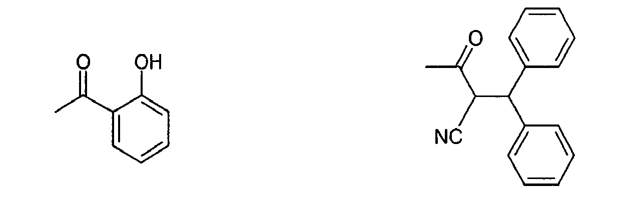 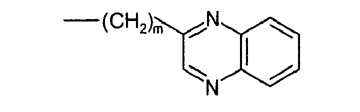 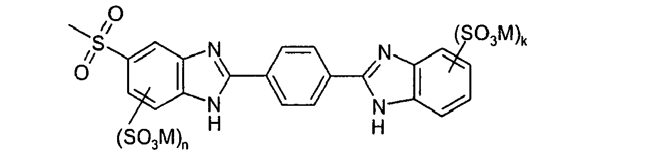 worin X, X₁, X₂ und X₃ jeweils unabhängig voneinander OH, CH₃COO, einen Alkoxyrest mit 1 bis 8 C-Atomen oder einen Monoglykosidrest bedeuten, n 0, 1, 2 oder 3, m 0 oder 1, k 0, 1, 2, 3 oder 4 und M H, Na oder K ist, und
worin ein oder mehrere Wasserstoffatome in den OH-Gruppen der in den Substituenten Z₁ bis Z₁₀ genannten Glykosidreste jeweils unabhängig voneinander auch durch Acetyl oder durch Alkylreste mit 1 bis 8 C-Atomen ersetzt sein können und wobei an ein oder mehrere Hydroxygruppen der in den Substituenten Z₁ bis Z₁₀ genannten Reste jeweils unabhängig voneinander auch Sulfat oder Phosphat gebunden sein kann.

Verbindungen der Formel I sind bereits bekannt. Allerdings wurden diese bisher nicht in kosmetischen Formulierungen verwendet.

Bekannte Verbindungen der Formel I sind z.B. Kaempferol 3-(6"-galloylglucosid) sowie Kaempferol 3-(6"-p-coumarylglucosid), welches auch als Tilirosid bezeichnet wird.

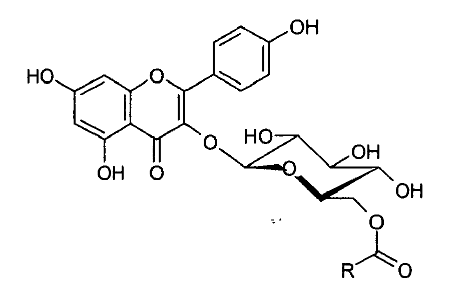

Kaempferol 3-(6"-galloylglucosid): R =

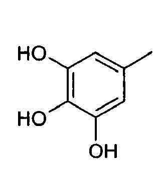

Tilirosid: R = 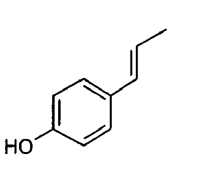 In der DE 195 44 905 A1 wird z.B. ein Verfahren zur Herstellung von Pflanzenextrakten enthaltend Tilirosid beschrieben sowie die Verwendung der Pflanzenextrakte in Arzneimitteln und Lebensmittelprodukten. Kosmetische Formulierungen enthaltend Tilirosid werden in der DE 195 44 905 A1 jedoch nicht beschrieben.

In der DE 199 22 287 A1 wird Tilirosid als ein Ausgangsflavonoid zur Herstellung von Tilirosidestern, deren Säureeinheit 3 bis 30 C-Atome enthält, beschrieben. Diese Ester werden in Kosmetika verwendet. In der DE 199 22 287 A1 werden jedoch keine kosmetischen Formulierungen enthaltend Tilirosid beschrieben.

Die erfindungsgemäßen kosmetischen Formulierungen enthaltend eine oder mehrere Verbindungen der Formel I besitzen beispielsweise den Vorteil, dass sie sowohl im UV-A- als auch im UV-B-Bereich absorbieren. Dies bedeutet, dass durch den Einsatz der erfindungsgemäßen Formulierungen ein Breitband-UV-Schutz erreicht werden kann. Zudem weisen die erfindungsgemäßen Formulierungen gute Absorptionskoeffizienten auf. Dies wird im folgenden am Beispiel der Substanz Tilirosid verdeutlicht.

Tilirosid absorbiert im UV-B-Bereich, also in dem Wellenlängenbereich zwischen 290 und 320 nm, mit einem Absorptionsmaximum bei λₘₐₓ = 316 nm und zusätzlich im UV-A-Bereich, also in dem Wellenlängenbereich zwischen 320 und 400 nm, mit einer Absorptionsschulter bei λₛₕ = 349 nm. Der Absorpionskoeffizient ε bei λₘₐₓ = 316 nm ist s = 23260 und bei λₛₕ = 349 nm ist ε = 13500.

Die vorteilhaften UV-absorbierenden Eigenschaften der in den erfindungsgemäßen kosmetischen Formulierungen enthaltenen Verbindungen der Formel I werden insbesondere deutlich, wenn man sie mit denen anderer Substanzen vergleicht, die im Handel erhältlich sind und in Sonnenschutzformulierungen verwendet werden. Beispielsweise zeigt Eusolex^{®} 6300 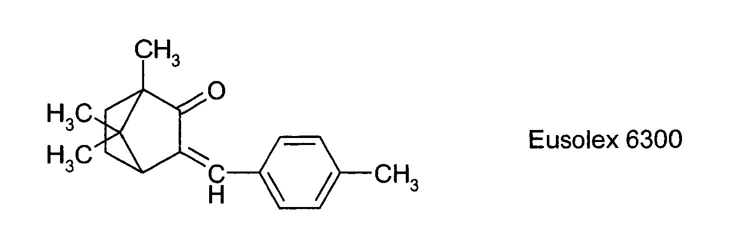 im UV-B-Bereich ein Absorptionsmaximum bei λₘₐₓ = 300 nm. Der Absorptionskoeffizient bei dieser Wellenlänge ist 23420. Eusolex^{®} 6300 absorbiert jedoch nicht im UV-A-Bereich. Dies bedeutet, dass Tilirosid und Eusolex^{®} 6300 im UV-B-Bereich einen vergleichbaren Absorptionskoeffizienten besitzen, während Tilirosid im UVA-Bereich signifikant bessere Absorptionseigenschaften aufweist.

Gegenstand der vorliegenden Erfindung ist daher auch die Verwendung von einer oder mehreren Verbindungen der Formel I als UV-Filter, insbesondere in kosmetischen Formulierungen.

Neben den vorteilhaften UV-absorbierenden Eigenschaften besitzen die Verbindungen der Formel I zudem vorteilhafte antioxidierende und radikalfangende Eigenschaften. Weiterhin Gegenstand der vorliegenden Erfindung ist somit auch die Verwendung von einer oder mehreren Verbindungen der Formel I als Radikalfänger und/oder Antioxidans, insbesondere in kosmetischen Formulierungen.

Durch ihre Wirkung als Antioxidans wirken Verbindungen der Formel I auch stabilisierend auf Formulierungen, die z.B. in der Kosmetik verwendet werden. Durch Beigabe von Verbindungen der Formel I zu den entsprechenden Produkten bleiben diese daher länger stabil und verändern ihr Aussehen nicht. Insbesondere bleibt auch bei länger dauernder Anwendung bzw. längerer Lagerung die Wirksamkeit der Inhaltsstoffe erhalten. Dies ist besonders vorteilhaft bei Sonnenschutzmitteln, da diese Kosmetika besonders hohen Belastungen durch UV-Strahlen ausgesetzt sind.

Die Formulierungen enthaltend eine oder mehrere Verbindungen der Formel I eignen sich besonders zum Schutz menschlicher Haut bzw. zum Schutz von Körperzellen gegen oxidativen Stress, d.h. z.B. gegen Schädigungen durch Radikale, wie sie z.B. durch Sonneneinstrahlung, Wärme oder andere Einflüsse erzeugt werden. Die Formulierungen enthaltend eine oder mehrere Verbindungen der Formel I sind insbesondere zur Verringerung der Hautalterung geeignet.

Gegenstand der vorliegenden Erfindung ist somit auch die Verwendung von einer oder mehreren Verbindungen der Formel I als Wirkstoff zum Schutz gegen oxidativen Stress, insbesondere in kosmetischen Formulierungen. Ein weiterer Gegenstand der vorliegenden Erfindung ist zudem die Verwendung von einer oder mehreren Verbindungen der Formel I zur Vermeidung der Hautalterung, insbesondere in kosmetischen Formulierungen.

Die Verbindungen der Formel I besitzen zudem anti-allergische, anti-inflammatorische, entzündungshemmende und anti-irritative Eigenschaften und können somit zur Behandlung oder vorbeugenden Behandlung von Allergien, Entzündungen und Irritationen, insbesondere der Haut, verwendet werden. Ein weiterer Gegenstand der vorliegenden Erfindung ist daher die Verwendung von einer oder mehreren Verbindungen der Formel I als Wirkstoff mit anti-allergischer, anti-inflammatorischer, entzündungshemmender und anti-irritativer Wirkung, insbesondere in kosmetischen Formulierungen.

Desweiteren besitzen Verbindungen der Formel I wie z.B. Tilirosid nur eine schwach ausgeprägte Eigenfarbe. Die schwach ausgeprägte Eigenfarbe ist z.B. dann von großem Vorteil, wenn in den Produkten eine Eigenfarbe der Inhaltsstoffe aus ästhetischen Gründen unerwünscht ist.

In den Verbindungen der Formel I sind die Alkoxygruppen vorzugsweise linear und besitzen 1 bis 12 und vorzugsweise 1 bis 8 C-Atome. Diese Gruppen entsprechen somit den Formeln -O-(CH₂)ₘ-H, wobei m 1, 2, 3, 4, 5, 6, 7 oder 8 und insbesondere 1 bis 5 bedeutet.

In den Verbindungen der Formel I sind die Hydroxyalkoxygruppen vorzugsweise linear und besitzen 2 bis 12 und vorzugsweise 2 bis 8 C-Atome. Diese Gruppen entsprechen somit den Formeln -O-(CH₂)ₙ-OH, wobei n 2, 3, 4, 5, 6, 7 oder 8, insbesondere 2 bis 5 und außerordentlich bevorzugt 2 bedeutet.

Falls einer oder mehrere der Reste Z₁ bis Z₄ und Z₆ bis Z₁₀ in den Verbindungen der Formel I einen Mono- oder Oligoglykosidrest bedeuten, so ist dieser Glykosidrest über ein Sauerstoffatom direkt an den entsprechenden Benzolring in Formel I gebunden. Die Mono- oder Oligoglykosidreste sind vorzugsweise aus 1 bis 3 Glykosideinheiten aufgebaut. Vorzugsweise sind diese Einheiten ausgewählt aus der Gruppe der Hexosylreste insbesondere der Rhamnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch erfindungsgemäß vorteilhaft sein, Pentosylreste zu verwenden.

Die in dem Rest Z₅ der Verbindungen der Formel I enthaltenen Mono- oder Oligoglykosidreste sind über ein Sauerstoffatom an die Gruppe "B" der Formel I gebunden und vorzugsweise aus 1 bis 3 Glykosideinheiten aufgebaut. Die bei den Resten Z₁ bis Z₄ und Z₆ bis Z₁₀ bevorzugten Einheiten sind auch für den in Rest Z₅ enthaltenen Mono- oder Oligoglykosidrest bevorzugt. Insbesondere bevorzugt ist der in dem Rest Z₅ enthaltene Mono- oder Oligoglykosidrest ausgewählt aus der Gruppe bestehend aus den Resten von Glucose, Rhamnose und Rutinose.

Falls X, X₁, X₂ und/oder X₃ in den Verbindungen der Formel I einen Monoglykosidrest bedeuten, sind diese Glykosidreste jeweils über ein Sauerstoffatom an den entsprechenden Benzolring gebunden. Die bei den Resten Z₁ bis Z₄ und Z₆ bis Z₁₀ bevorzugten Einheiten sind auch für diesen Monoglykosidrest bevorzugt. Falls X, X₁, X₂ und/oder X₃ einen Monoglykosidrest bedeuten, ist der Glucoserest besonders bevorzugt.

In einer bevorzugten Ausführungsform der Erfindung, insbesondere wenn die Wasserlöslichkeit der Verbindungen der Formel I gesteigert werden soll, ist an eine oder an mehrere Hydroxygruppen der in den Substituenten Z₁ bis Z₁₀ genannten Reste eine polare Gruppe gebunden, z.B. jeweils unabhängig voneinander eine Sulfat- oder Phosphatgruppe. Geeignete Gegenionen sind beispielsweise die Ionen der Alkali- oder Erdalkalimetalle, wobei diese z.B. aus Natrium oder Kalium ausgewählt sind.

In einer weiteren bevorzugten Ausführungsform der Erfindung sind diejenigen Verbindungen der Formel I bevorzugt, in denen die in dem Substituenten Z₅ enthaltenen Reste mit aromatischer Komponente über eine Estergruppe -OOC- an den ebenfalls im Rest Z₅ enthaltenen Mono-oder Oligoglykosidrest gebunden sind.

In einer weiteren bevorzugten Ausführungsform der Erfindung leiten sich Teilformeln der Formel I von den Verbindungen der nachstehenden Gruppe ab: Rutin, Trishydroxyethylrutin (Troxerutin), Isoquercetin, Trishydroxyethylisoquercetin (Troxeisoquercetin) und Astragalin sowie deren Sulfate und Phosphate.

In einer weiteren bevorzugten Ausführungsform sind die in den erfindungsgemässen Formulierungen enthaltenen Verbindungen der Formel I ausgewählt aus den Verbindungen der Formel IA 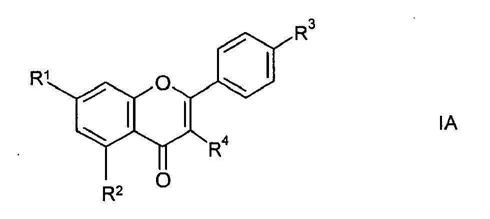 worin
R¹, R² und R³ jeweils unabhängig voneinander OH, CH₃COO, einen Alkoxyrest mit 1 bis 8C-Atomen oder einen Monoglykosidrest bedeuten,
R⁴ ein Mono- oder Diglykosidrest ist, wobei an den Glykosidrest jeweils über eine Gruppe -O- mindestens eine Gruppe ausgewählt aus 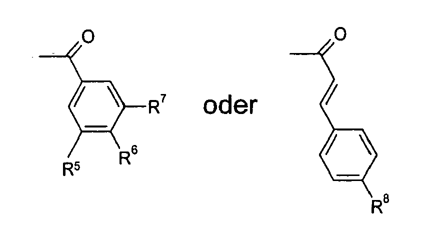 gebunden ist,
R⁵, R⁶, R⁷ und R⁸
jeweils unabhängig voneinander die Bedeutung der Reste R¹ bis R³ besitzen, und worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann.

In einer bevorzugten Ausführungsform ist der Rest R² in den Verbindungen der Formel IA ausgewählt aus OH, CH₃COO oder einem Alkoxyrest mit 1 bis 8 C-Atomen.

In den Verbindungen der Formel IA können alle OH-Gruppen des Mono-oder Diglykosidrests von R⁴ mit einer Gruppe der Formel 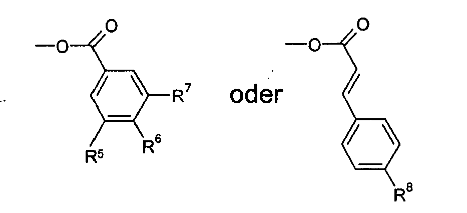 verestert sein. Vorzugsweise sind jedoch nur ein oder zwei der von diesen Resten abgeleiteten Reste an den Glykosidrest gebunden.

Wenn R⁴ ein Mono- oder Diglykosidrest ist, in dem ein oder mehrere Wasserstoffatome der OH-Gruppen durch Acetyl oder durch Alkylreste ersetzt sind, dann sind vorzugsweise alle OH-Gruppen, für die der Ersatz möglich ist, durch Acetyl oder durch Alkyl ersetzt.

Unter den in den Verbindungen der Formel IA genannten Alkoxyresten mit 1 bis 8 C-Atomen ist die Methoxygruppe bevorzugt. Unter den in den Verbindungen der Formel IA genannten Alkylresten mit 1 bis 8 C-Atomen ist die Methylgruppe bevorzugt.

Die in den Verbindungen der Formel lA genannten Mono- und Diglykosidreste sind vorzugsweise aus Glucoseeinheiten aufgebaut.

lm folgenden werden bevorzugte Verbindungen IA1 bis IA13 ausgewählt aus den Verbindungen der Formel lA angegeben: 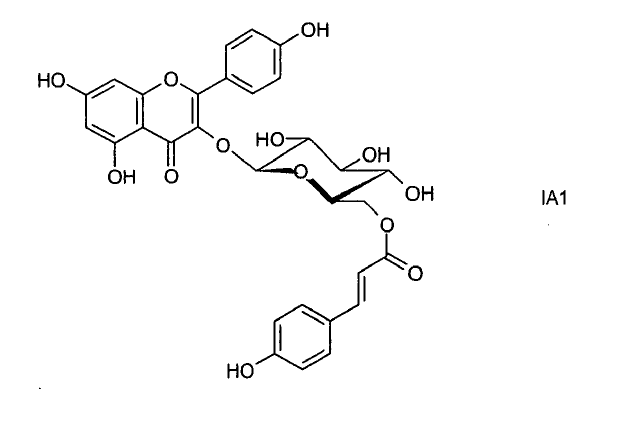 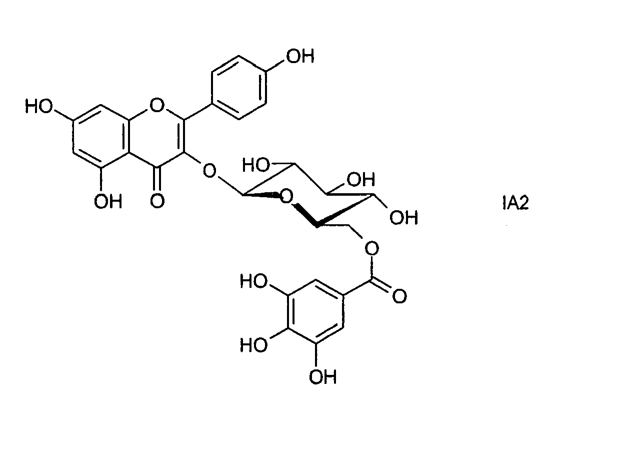 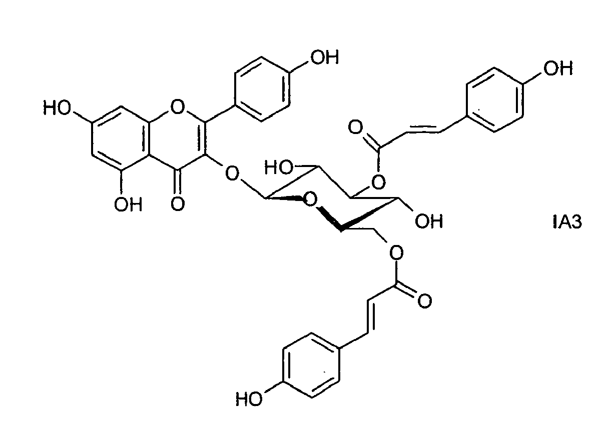 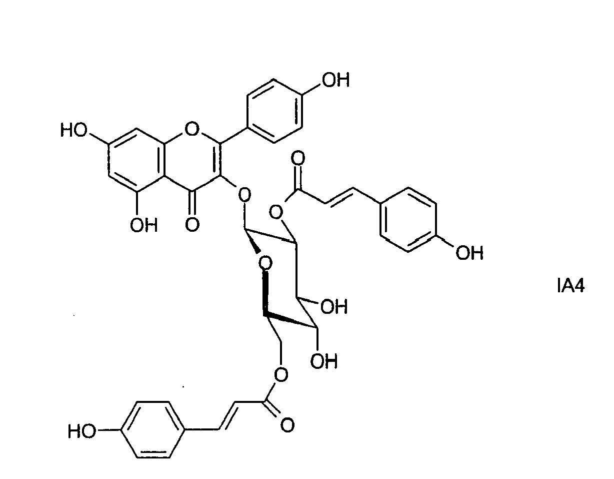 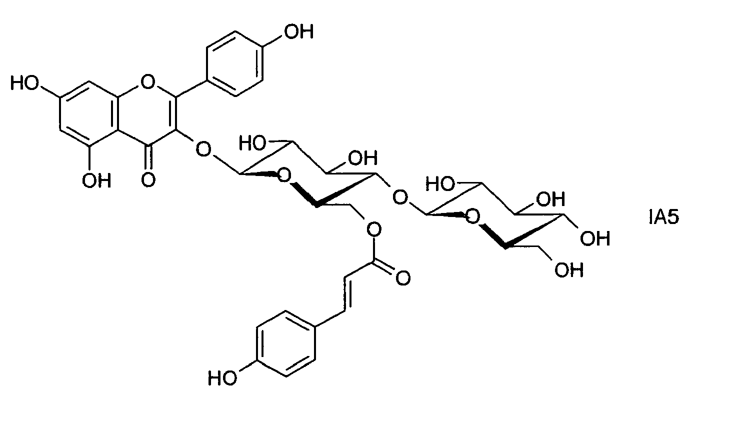 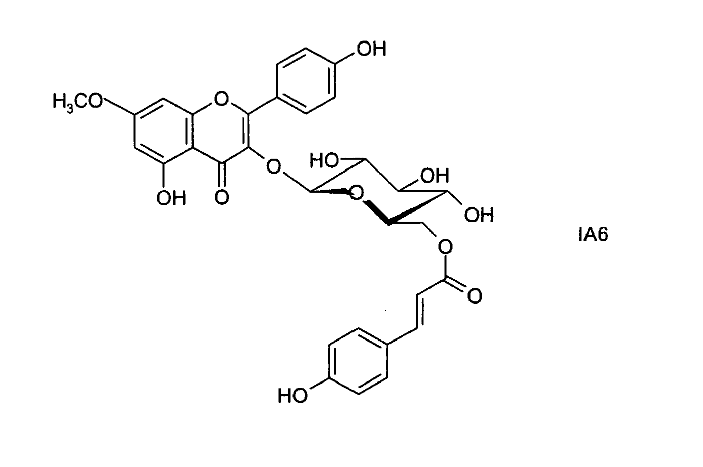 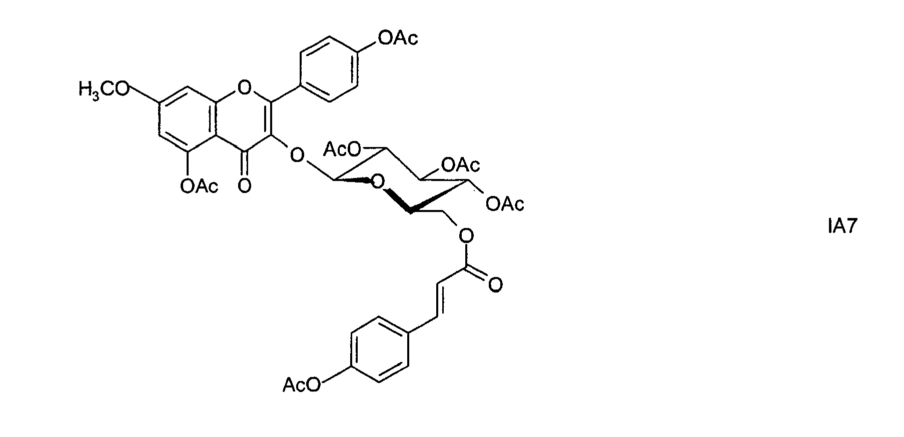 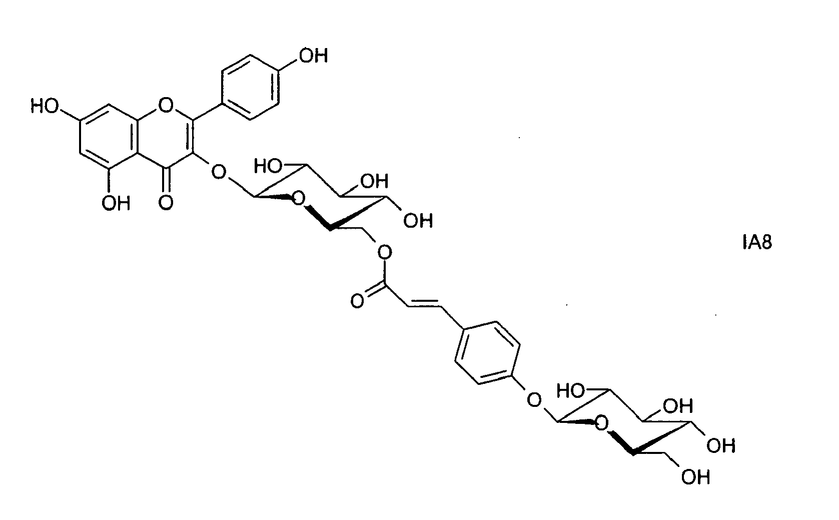 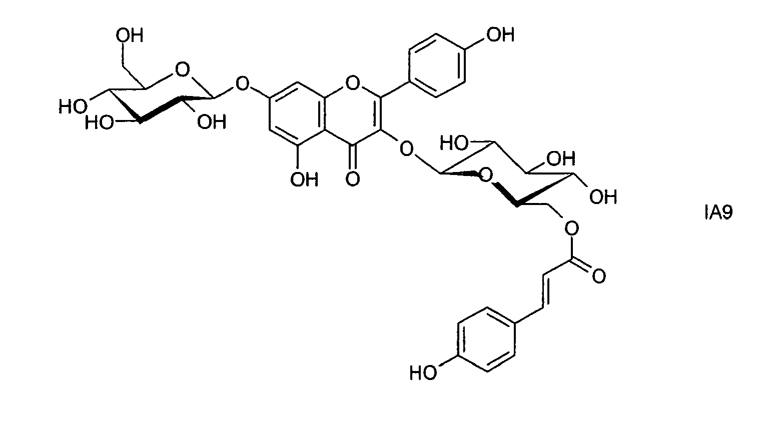 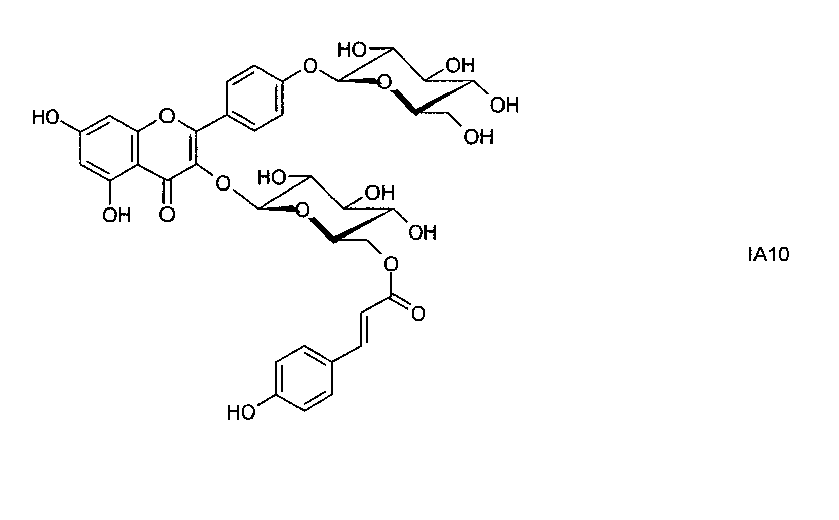 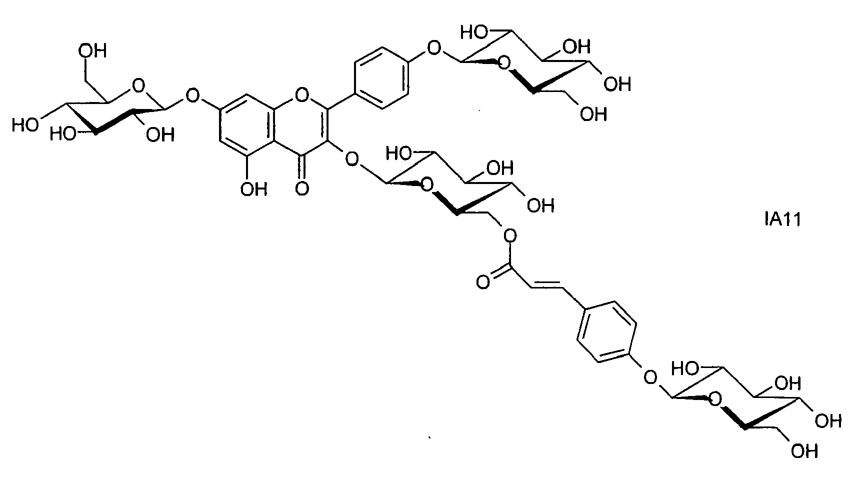 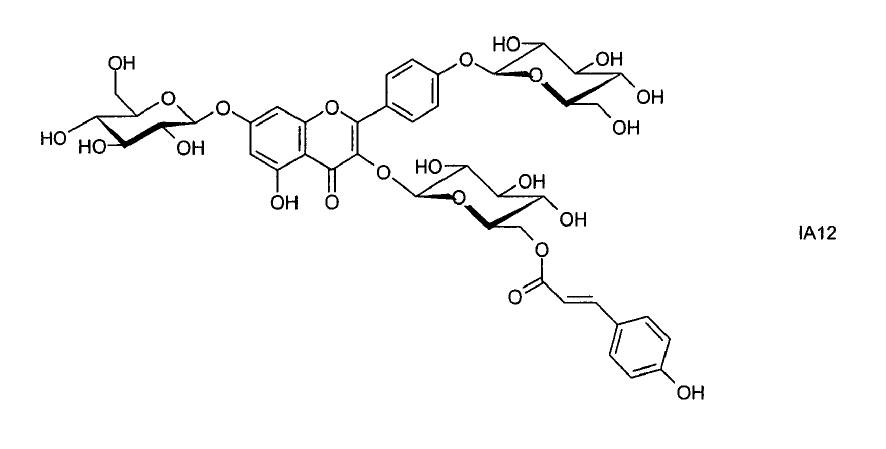 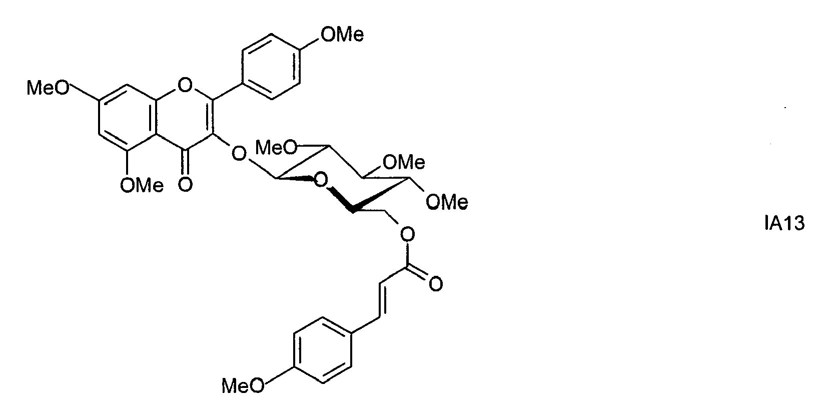 In den oben aufgeführten Verbindungen der Formeln IA1 bis lA13 bedeuten Me Methyl und Ac Acetyl.

Unter den Verbindungen der Formel IA sind insbesondere die Verbindungen der Formlein IA1 und IA2 bevorzugt. Ganz ausserordentlich bevorzugt ist die Verbindung der Formel IA1, also Tilirosid.

In einer weiteren bevorzugten Ausführungsform sind die in den erfindungsgemässen Formulierungen enthaltenen Verbindungen der Formel I ausgewählt aus den Verbindungen, worin
Z₁ bis Z₄ und Z₆ bis Z₁₀ jeweils unabhängig voneinander H, OH, Alkoxy, Hydroxyalkoxy, Mono- oder Oligoglykosidreste bedeuten und wobei die Alkoxy- und Hydroxyalkoxygruppen verzweigt und unverzweigt sein und 1 bis 18 C-Atome aufweisen können, 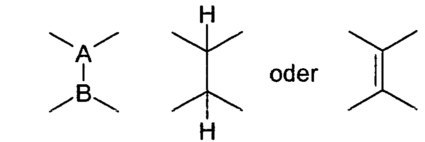 bedeutet,
Z₅, n, m, k und M die in Anspruch 1 genannten Bedeutungen besitzen, aber die in dem Substituenten Z₅ enthaltenen Reste X, X₁, X₂ und X₃ jeweils unabhängig voneinander OH, einen Alkoxyrest mit 1 bis 8 C-Atomen oder einen Monoglykosidrest bedeuten,
und worin ein oder mehrere Wasserstoffatome in den OH-Gruppen der in den Substituenten Z₁ bis Z₁₀ genannten Glykosidreste jeweils unabhängig voneinander auch durch Alkylreste mit 1 bis 8 C-Atomen ersetzt sein können und wobei an ein oder mehrere Hydroxygruppen der in den Substituenten Z₁ bis Z₁₀ genannten Reste jeweils unabhängig voneinander auch Sulfat oder Phosphat gebunden sein kann.

In diesen Verbindungen der Formel I bedeuten Z₁ bis Z₄ und Z₆ bis Z₁₀ vorzugsweise jeweils unabhängig voneinander H, OH, Alkoxy oder Hydroxyalkoxy.

In einer weiteren bevorzugten Ausführungsform sind die in den erfindungsgemässen Formulierungen enthaltenen Verbindungen der Formel IA ausgewählt aus den Verbindungen, worin
R¹, R² und R³ jeweils unabhängig voneinander OH, einen Alkoxyrest mit 1 bis 8 C-Atomen oder einen Monoglykosidrest bedeuten,
R⁴ ein Mono- oder Diglykosidrest ist, wobei an den Glykosidrest jeweils über eine Gruppe -O- mindestens eine Gruppe ausgewählt aus 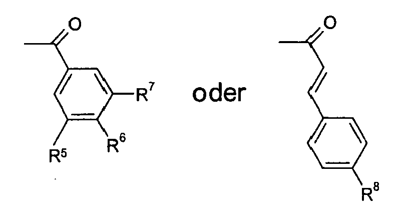 gebunden ist,
R⁵, R⁶ R⁷ und R⁸
jeweils unabhängig voneinander OH, einen Alkoxyrest mit 1 bis 8 C-Atomen oder einen Monoglykosidrest bedeuten, und
worin ein oder mehrere Wasserstoffatome in den OH-Gruppen des oder der Glykosidreste jeweils unabhängig voneinander auch durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann.

In diesen Verbindungen der Formel lA bedeuten R¹ bis R³ vorzugsweise jeweils unabhängig voneinander OH oder einen Alkoxyrest mit 1 bis 8 C-Atomen.

Einige Verbindungen der Formel I wie z.B. Tilirosid können aus Pflanzen gewonnen werden, z.B. aus den Pflanzen der Gattung Althaea, Aristolochia, Helianthemum, Lindera, Magnolia, Platanus, Potentilla, Quercus, Rosa, Sida, Sorbus und/oder Tilia. Diese Verbindungen können entweder in isolierter Form oder auch in nicht isolierter Form weiterverarbeitet werden, also z.B. in Form eines Extrakts oder in Form eines aufgereinigten Extrakts oder auch in Form der aus dem Pflanzenextrakt hergestellten Reinsubstanz in kosmetische Formulierungen eingearbeitet werden. Unter den genannten Gattungen sind folgende Arten bevorzugt: Althaea officinalis, Althaea rosea, Aristolochia heterophylla, Helianthemum glomeratum, Lindera megaphylla, Magnolia salicifolia, Platanus acerifolia, Platanus occidentalis, Potentilla anserina, Quercus pubescens, Quercus suber, Quercus laurifolia, Quercus ilex, Quercus imbricaria, Quercus virginiana, Rosa pomifera, Sida rhombifolia, Sida poeppigiana, Sida cordifolia, Sida glaziovii, Sorbus pendula, Tilia argenta und Tilia cordata.

Wenn die erfindungsgemäße kosmetische Formulierung Tilirosid enthält, ist diese Verbindung in einer weiteren bevorzugten Ausführungsform in der Form eines Pflanzenextrakts, eines aufgereinigten Pflanzenextrakts oder in Form der aus dem Pflanzenextrakt hergestellten Reinsubstanz zur Herstellung der kosmetischen Formulierung verwendet worden. In derartigen kosmetischen Formulierungen enthält der Pflanzenextrakt beispielsweise 1 bis 100 Gew.% Tilirosid. In einer Ausführungsform enthält der Pflanzenextrakt vorzugsweise 5 bis 90 Gew.% Tilirosid. ln einer weiteren Ausführungsform enthält der Pflanzenextrakt vorzugsweise 30 bis 100 Gew.%, besonders bevorzugt 60 bis 100 Gew.% und insbesondere bevorzugt 90 bis 100 Gew.% Tilirosid. In einer weiteren bevorzugten Ausführungsform ist der Pflanzenextrakt durch Extraktion der Pflanze Sida glaziovii gewonnen worden.

Bei allen erfindungsgemäßen Verwendungen, in denen Tilirosid zum Einsatz kommt, z.B. wenn Tilirosid als UV-Filter, als Radikalfänger und/oder Antioxidans, gegen oxidativen Stress, zur Vermeidung der Hautalterung oder als Wirkstoff mit anti-allergischer, anti-inflammatorischer, entzündungshemmender oder anti-irritativer Wirkung eingesetzt wird, insbesondere in kosmetischen Formulierungen, kann Tilirosid z.B. in Form einer synthetisch gewonnenen Substanz, in Form eines Pflanzenextrakts, eines aufgereinigten Pflanzenextrakts oder als Einzelsubstanz bzw. in Form einer aus dem Pflanzenextrakt gewonnenen Reinsubstanz verwendet werden. ln einer bevorzugten Ausführungsform wird Tilirosid hierbei in der Form eines Pflanzenextrakts, eines aufgereinigten Pflanzenextrakts oder in Form der aus dem Pflanzenextrakt hergestellten Reinsubstanz verwendet.

Die Verbindungen der Formel I können nach Methoden, die dem Fachmann wohl bekannt und in der Literatur beschrieben sind (z.B. in Standard-Werken wie Houben-Weyl, Methoden der organischen Chemie, Georg-Thieme-Verlag, Stuttgart), gewonnen oder hergestellt werden.

Beispielsweise kommt Tilirosid in Pflanzen vor und kann durch Extraktion gewonnen werden. Die Herstellung der Pflanzenextrakte erfolgt durch übliche Methoden der Extraktion der Pflanzen bzw. Pflanzenteile. Geeignete Extraktionsverfahren können sein: Mazeration, Remazeration, Digestion, Bewegungsmazeration, Wirbelextraktion, Ultraschallextraktion, Gegenstromextraktion, Perkolation, Reperkolation, Evakolation, Diakolation oder Festflüssig-Extraktion unter kontinuierlichem Rückfluss, die in einem Soxhlet-Extraktor durchgeführt wird.

Als Lösungsmittel für die Extraktion kann beispielsweise Wasser oder ein Alkohol verwendet werden.

Es ist dem allgemeinen Wissen des Fachmanns zuzurechnen, wie diese Extraktionen im Einzelnen durchgeführt werden und die erhaltenen Rohextrakte durch allgemein geläufige Methoden aufgereinigt werden können.

Ein möglicher Syntheseweg für Tilirosid ist z.B. auch in B. Vermes, H. Wagner, Stud. Org. Chem. (Amsterdam) (1982), Volume date 1981, 11 (Flavonoids, Bioflavonoids), 161-167 und in B. Vermes, V.M. Chari, H. Wagner, Helv. Chim. Acta (1981), 64(4), 1964-1967 beschrieben.

Die Synthese von Tilirosid ist in Schema 1 wiedergegeben. 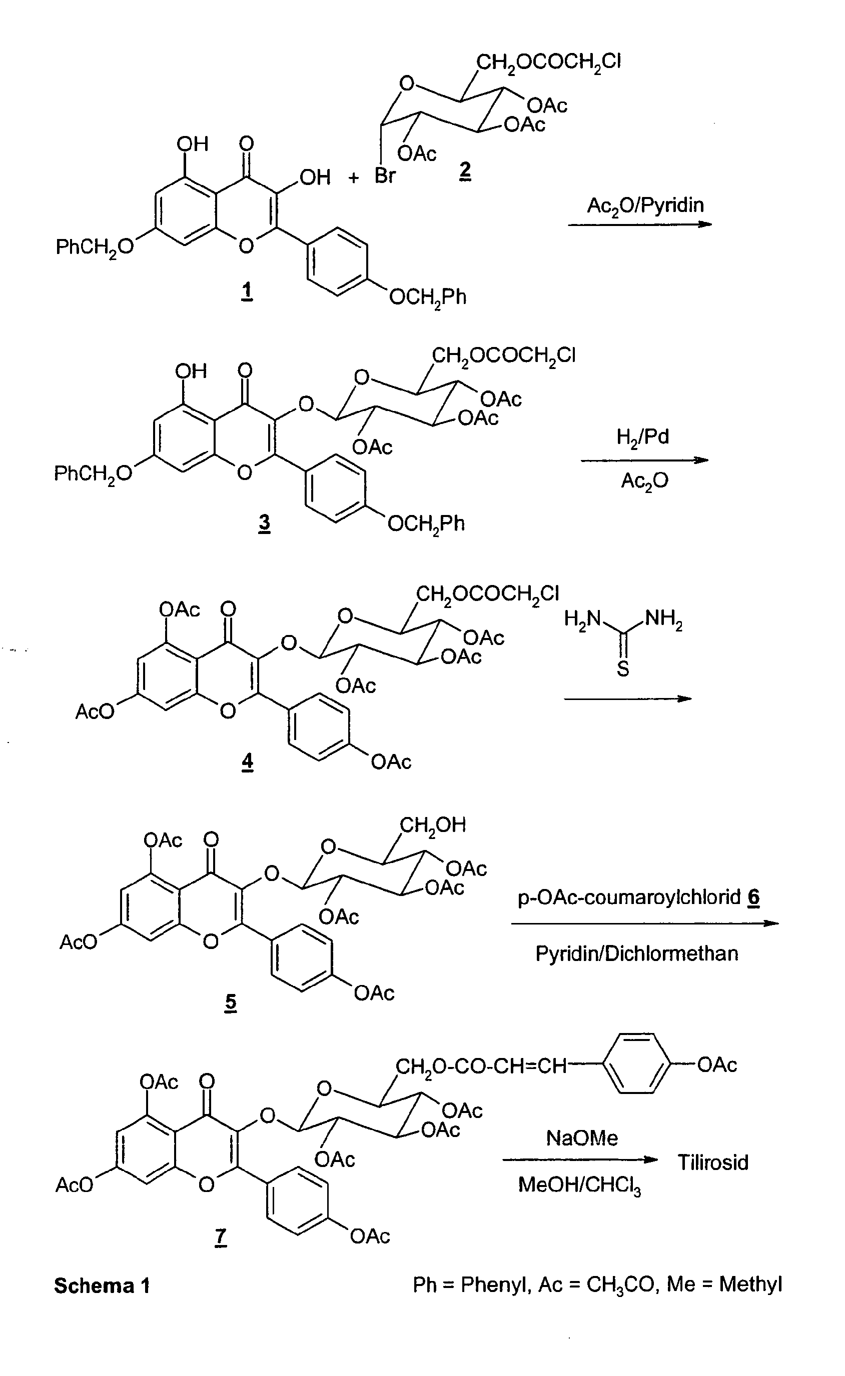 4',7-Dibenzylkaempferol (1) [H. Wagner, H. Danninger, O. Seligmann, M. Nógrádi, L. Farkas, N. Farnsworth, Chem. Ber. 103 (1978) 3768] wird in Gegenwart von Ag₂CO₃ und Pyridin mit 2,3,4-Tri-O-acetyl-6-O-chloroacetyl-β-D-glucopyranosylbromid **(2)** zu Verbindung **3** umgesetzt. Die Verbindung **2** kann nach der in D.Y. Gagniere, P.J.A. Wottero, Carbohydrate Res. 28 (1973) 1965 beschriebenen Methode hergestellt werden. Die katalytische Debenzylierung und anschließende vorsichtige Acetylierung der Verbindung **3** liefert Verbindung **4** aus der nach Entfernung der Chloracetyl-Gruppe mit Thioharnstoff Verbindung **5** erhalten werden kann. In dieser Verbindung ist lediglich eine Hydroxylgruppe frei, sodaß die Veresterung der Verbindung **5** selektiv verlaufen kann. Die Veresterung mit dem Säurechlorid p-Acetylcoumaroylchlorid **6** kann in einer Mischung aus Pyridin und Dichloromethan durchgeführt werden. Damit die Veresterung vollständig abläuft ist ein Überschuß an Säurechlorid und eine lange Reaktionszeit (ca. 96h) bei Raumtemperatur notwendig. Der letzte Schritt, die selektive Verseifung der 7 Acetylgruppen in Verbindung **7**, kann nach der in G. Zemplén, Chem. Ber. 59 (1926) 1258 beschriebenen Methode durchgeführt werden. Hierbei wird unter Verwendung einer katalytischen Menge an NaOCH₃ und einer kalkulierten Menge an Methanol gearbeitet.

Andere Verbindungen der Formel I können durch routinemäßige Abwandlung der in Schema 1 gezeigten Synthese erhalten werden. Hierbei werden je nach Zielmolekül andere Edukte, d.h. andere gegebenenfalls geschützte Flavonoide, Zuckerkomponenten und Reste, die an die Zuckerkomponente angehängt werden sollen, verwendet.

Die Veresterung glykosidischer OH-Gruppen mit aromatischen Sulfonsäure-Einheiten kann beispielsweise nach der in A.B. Foster et al., J. Chem. Soc. (1954) 3625-3629 beschriebenen Methode erfolgen. Hiernach kann die Zuckerkomponente z.B. mit einem entsprechenden aromatischen Sulfonsäurechlorid in Pyridin zur Reaktion gebracht werden.

Die Veretherung glykosidischer OH-Gruppen mit aromatischen Resten kann beispielsweise nach der in P. Beraud et al., Tetrahedron Let. 30(3) (1989) 325-326 beschriebenen Methode erfolgen. Bei dieser Mitsunobu-Reaktion findet die Veretherung beispielsweise derart statt, dass die Zuckerkomponente zusammen mit Triphenylphosphin PPh₃ in Pyridin gelöst und mit einer entsprecheneden Phenolkomponente und Diethylazodicarboxylat zur Reaktion gebracht wird.

Die Veretherung glykosidischer OH-Gruppen mit Resten gesättigter Kohlenwasserstoffe kann beispielsweise nach der in M. Goebel et al., Tetrahedron 53(9) (1997) 3123-3134 beschriebenen Methode erfolgen. Die Veretherung findet z.B. derart statt, dass die Zuckerkomponente in trockenem Dimethylformamid unter Inertgas vorsichtig mit Natriumhydrid versetzt und danach mit einem geeigneten Alkylierungsreagenz wie z.B. einem entsprechenden Bromid vorsichtig umgesetzt wird.

Der Anteil der Verbindungen der Formel I in der kosmetischen Formulierung beträgt vorzugsweise von 0,001 bis 20 Gew.%, besonders bevorzugt von 0,01 bis 10 Gew.% und insbesondere bevorzugt von 0,05 bis 5 Gew.% bezogen auf die gesamte kosmetische Formulierung. Ganz außerordentlich bevorzugt beträgt der Anteil der Verbindungen der Formel I in der kosmetischen Formulierung von 0,05 bis 2 Gew.% bezogen auf die gesamte kosmetische Formulierung.

Die schützende Wirkung der erfindungsgemäßen kosmetischen Formulierungen gegen UV-Strahlung kann verbessert werden, wenn die Formulierung neben den Verbindungen der Formel I einen oder mehrere weitere UV-Filter enthält.

Prinzipiell kommen alle UV-Filter für eine Kombination in Frage. Besonders bevorzugt sind solche UV-Filter, deren physiologische Unbedenklichkeit bereits nachgewiesen ist. Sowohl für UV-A- als auch UVB-Filter gibt es viele aus der Fachliteratur bekannte und bewährte Substanzen, z.B.

Benzylidenkampferderivate wie
- 3-(4'-Methylbenzyliden)-dl-kampfer (z.B. Eusolex® 6300),
- 3-Benzylidenkampfer (z.B. Mexoryl^{®} SD),
- Polymere von N-{(2 und 4)-[(2-oxoborn-3-yliden)methyl]benzyl}-acrylamid (z.B. Mexoryl^{®} SW),
- N,N,N-Trimethyl-4-(2-oxoborn-3-ylidenmethyl)anilinium methylsulfat (z.B. Mexoryl^{®} SK) oder
- α-(2-Oxoborn-3-yliden)toluol-4-sulfonsäure (z.B. Mexoryl^{®} SL),

Benzoyl- oder Dibenzoylmethane wie
- 1-(4-tert-Butylphenyl)-3-(4-methoxyphenyl)propan-1,3-dion (z.B. Eusolex® 9020) oder
- 4-Isopropyldibenzoylmethan (z.B. Eusolex® 8020),

Benzophenone wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),

Methoxyzimtsäureester wie
- Methoxyzimtsäureoctylester (z.B. Eusolex^{®} 2292),
- 4-Methoxyzimtsäureisopentylester, z.B. als Gemisch der Isomere (z.B. Neo Heliopan^{®} E 1000),

Salicylatderivate wie
- 2-Ethylhexylsalicylat (z.B. Eusolex^{®} OS),
- 4-Isopropylbenzylsalicylat (z.B. Megasol^{®}) oder
- 3,3,5-Trimethylcyclohexylsalicylat (z.B. Eusolex^{®} HMS),

4-Aminobenzoesäure und Derivate wie
- 4-Aminobenzoesäure,
- 4-(Dimethylamino)benzoesäure-2-ethylhexylester (z.B. Eusolex^{®} 6007),
- ethoxylierter 4-Aminobenzoesäureethylester (z.B. Uvinul^{®} P25),

Benzimidazolderivate wie
- 2-Phenylbenzimidazol-5-sulfonsäure sowie ihre Kalium-, Natrium- und Triethanolaminsalze (z.B. Eusolex^{®} 232),
- 2,2'-(1,4-Phenylen)bis-(1H-benzimidazol-4,6-disulfonsäure, Mononatriumsalz) (CAS-Nr. 180 898-37-7),
- 2,2'-(1,4-Phenylen)bis-(1 H-benzimidazol-5-sulfonsäure) sowie ihre Kalium-, Natrium- und Triethanolaminsalze, und weitere Substanzen wie
- 2-Cyano-3,3-diphenylacrylsäure-2-ethylhexylester (z.B. Eusolex® OCR),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl^{®} SX),
- 2,4,6-Trianilino-(p-carbo-2'-ethylhexyl-1'-oxi)-1,3,5-triazin (z.B. Uvinul^{®} T 150),
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)-propenyl) und 0,1 bis 0,4% (methylhydrogen]silylen]] (n ≈ 60) (CAS-Nr. 207 574-74-1),
- 2,2'-Methylen-bis-(6-(2H-benzotriazol-2-yl)-4-(1,1,3,3-tetramethylbutyl)phenol) (CAS-Nr. 103 597-45-1),
- 2,4-bis-{[4-(2-Ethyl-hexyloxy)-2-hydroxyl]-phenyl}-6-(4-methoxyphenyl)-1,3,5-triazin (CAS-Nr. 103 597-45-, 187 393-00-6).

Die in der Liste aufgeführten Verbindungen sind nur als Beispiele aufzufassen. Selbstverständlich können auch andere UV-Filter verwendet werden. Diese organischen UV-Filter werden wie auch die Verbindungen der Formel I in der Regel in einer Menge von 0,5 bis 20 Gew.-%, vorzugsweise in einer Menge von 1 bis 15 Gew.-% und insbesondere bevorzugt in Mengen von 2 bis 8 Gew.-% je Einzelsubstanz in kosmetische Formulierungen eingearbeitet. Insgesamt enthalten die kosmetischen Zubereitungen üblicherweise bis zu 40 Gew.-%, vorzugsweise 5 bis 25 Gew.-% solcher organischer UV-Filter.

Als anorganische UV-Filter sind solche aus der Gruppe der Titandioxide wie z.B. gecoatetes Titandioxid (z.B. Eusolex^{®} T-2000, Eusolex^{®} T-AQUA), Zinkoxide (z.B. Sachtotec^{®}), Eisenoxide oder auch Ceroxide denkbar. Diese anorganischen UV-Filter werden in der Regel in einer Menge von 0,5 bis 20 Gew.-%, vorzugsweise 2 bis 10 Gew.-%, in kosmetische Formulierungen eingearbeitet.

Werden verschiedene anorganische oder organische UV-Filter eingesetzt, so können diese in nahezu beliebigen Verhältnissen zueinander verwendet werden. Üblicherweise liegen die Verhältnisse der einzelnen Substanzen zueinander im Bereich 1:10 - 10:1, vorzugsweise im Bereich 1:5 - 5:1 und insbesondere bevorzugt im Bereich 1:2 - 2:1. Werden UV-A- neben UV-B-Filtern eingesetzt, so ist es für die meisten Anwendungen von Vorteil, wenn der Anteil an UV-B-Filtern überwiegt und das Verhältnis von UV-A-Filtern : UV-B-Filtern im Bereich 1:1 bis 1:10 liegt.

Neben den Verbindungen der Formel I sind als bevorzugte Verbindungen mit UV-filternden Eigenschaften für die kosmetischen Zubereitungen 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester und gecoatetes Titandioxid zu nennen.

Die schützende Wirkung gegen oxidativen Stress bzw. gegen die Einwirkung von Radikalen kann weiter verbessert werden, wenn die Formulierung ein oder mehrere weitere Antioxidantien enthält.

Es gibt viele aus der Fachliteratur bekannte und bewährte Substanzen, die verwendet werden können, z.B. Aminosäuren (z.B. Glycin, Histidin, Tyrosin, Tryptophan) und deren Derivate, lmidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen kosmetischen Formulierungen geeignet. Bekannte und käufliche Mischungen sind beispielsweise Mischungen enthaltend als aktive Inhaltsstoffe Lecithin, L-(+)-Ascorbylpalmitat und Zitronensäure (z.B. (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-α-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004),

Der Anteil des einen oder der mehreren Antioxidantien in der kosmetischen Formulierung beträgt vorzugsweise von 0,001 bis 5 Gew.%, besonders bevorzugt von 0,01 bis 2 Gew.% bezogen auf die gesamte Formulierung.

Die schützende Wirkung der erfindungsgemäßen kosmetischen Formulierungen gegen UV-Strahlung und/oder oxidativen Stress kann auch verbessert werden, wenn die Formulierung neben den Verbindungen der Formel I ein oder mehrere Verbindungen ausgewählt aus Flavonoiden und Coumaranonen enthält. Als Flavonoide werden die Glykoside von Flavanonen, Flavonen, 3-Hydroxyflavonen (= Flavonolen), Auronen, Isoflavonen und Rotenoiden aufgefaßt [Römpp Chemie Lexikon, Band 9, 1993]. Im Rahmen der vorliegenden Erfindung werden hierunter jedoch auch die Aglykone, d.h. die zuckerfreien Bestandteile, und die Derivate der Flavonoide und der Aglykone verstanden. Weiterhin wird im Rahmen der vorliegenden Erfindung unter dem Begriff Flavonoid auch Anthocyanidin (Cyanidin) verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

Die Flavonoide sind vorzugsweise ausgewählt aus folgenden Verbindungen: 4,6,3',4'-Tetrahydroxyauron, Quercetin, Rutin, Isoquercetin, Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfaten und Phosphaten. Unter den Flavonoiden sind insbesondere Rutin und Troxerutin bevorzugt. Ganz außerordentlich bevorzugt ist Troxerutin.

Unter den Coumaranonen ist 4,6,3',4'-Tetrahydroxybenzylcoumaranon-3 bevorzugt.

Der Anteil der einen oder mehreren Verbindungen ausgewählt aus Flavonoiden und Coumaranonen in der kosmetischen Formulierung beträgt vorzugsweise von 0,001 bis 5 Gew.%, besonders bevorzugt von 0,01 bis 2 Gew.% bezogen auf die gesamte Formulierung.

Die erfindungsgemäßen Formulierungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin B₁), Riboflavin (Vitamin B₂), Nicotinsäureamid, Vitamin C (Ascorbinsäure), Vitamin D, Ergocalciferol (Vitamin D₂), Vitamin E, DL-α-Tocopherol, Tocopherol-E-Acetat, Tocopherolhydrogensuccinat, Vitamin K₁, Esculin (Vitamin P-Wirkstoff), Thiamin (Vitamin B₁), Nicotinsäure (Niacin), Pyridoxin, Pyridoxal, Pyridoxamin, (Vitamin B₆), Panthothensäure, Biotin, Folsäure und Cobalamin (Vitamin B₁₂) in den erfindungsgemäßen kosmetischen Formulierungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäßen Formulierungen können weiter als Inhaltsstoff auch Ectoin [(S)-1,4,5,6-Tetrahydro-2-methyl-4-pyrimidincarbonsäure] enthalten und bewirken dann einen Schutz von Zellen der Haut, insbesondere einen Schutz der Langerhanszellen. Besonders vorteilhaft sind kosmetische Formulierungen enthaltend Tilirosid und Ectoin.

Durch die Zugabe von 1-(2-Hydroxyaryl)-alkan-1-on-oximen (wie z.B. in der EP 0 149 242 beschrieben) und vorzugsweise von 2-Hydroxy-5-methyl-laurophenonoxim erhält die erfindungsgemäße Formulierung eine vorteilhafte anti-inflammatorische Wirkung. Besonders vorteilhaft sind kosmetische Formulierungen enthaltend Tilirosid und 2-Hydroxy-5-methyl-laurophenonoxim, worin die genannten Substanzen in einem Gewichtsverhältnis von 1:10 bis 10:1 1 enthalten sind. Anwendungsformen derartiger Formulierungen sind z.B. After-Sun-Präparate.

Weiterhin sind auch erfindungsgemäße Formulierungen bevorzugt, die Tilirosid und 4,6,3',4'-Tetrahydroxybenzylcoumaranon-3 enthalten. In diesen Formulierungen sind die genannten Substanzen in einem Gewichtsverhältnis von 1 : 10 bis 10 : 1 enthalten.

In die erfindungsgemäßen Formulierungen können auch weitere Wirkstoffe eingearbeitet werden, z.B.
- Hydroxyectoin [(S,S)-1,4,5,6-Tetrahydro-5-hydroxy-2-methyl-4-pyrimidincarbonsäure]
- Wirkstoffe, die zur Wundbehandlung dienen können, wie z.B. Allantoin
- Insekt-Repellentien wie z.B. 3-[N-n-butyl-N-acetyl]-aminopropionsäure-ethylester [CAS-Nr. 52304-36-6]
- Sorbit für die Hautpflege [z.B. Karion^{®}F flüssig oder Karion^{®}FP flüssig]
- Biotin
- anti-ageing-Produkte wie z.B. Mischungen enthaltend Hydroxyprolin oder Derivate von Hydroxyprolin, z.B. Mischungen enthaltend Lecithin, Hydroxyprolindipalmitat, Sitosterol, Linolsäure, Tocopherol, Natriumascorbat, Mannit, Phenoxyethanol, Methylparaben, Ethylparaben, Propylparaben, Butylparaben, Wasser [z.B. RonaCare^{™} ASC III^{®}] oder z.B. Mischungen enthaltend Lecithin, hydroxyliertes Lecithin, L-Hydroxyprolin, Dinatrium Rutinyldisulfat, Phenoxyethanol, Mannit, Magnesiumascorbylphosphat, Methylparaben, Ethylparaben, Propylparaben, Butylparaben, Sitosterol, Tocopherol, Natriumascorbat, Wasser [z.B. RonaCare^{™} VTA]
- Bisabolol.

Die Verbindungen der Formel I können in der üblichen Weise in kosmetische Formulierungen eingearbeitet werden. Geeignet sind Formulierungen für eine äußerliche Anwendung, beispielsweise als Creme, Lotion, Gel, oder als Lösung, die auf die Haut aufgesprüht werden kann. Dabei ist es bevorzugt, wenn die Zubereitung mindestens eine ÖI-und mindestens eine Wasser-Phase enthält.

Als Anwendungsform der erfindungsgemäßen kosmetischen Formulierungen seien z.B. genannt: Lösungen, Emulsionen, PIT-Emulsionen, Suspensionen, Pasten, Salben, Gele, Cremes, Seifen, tensidhaltige Reinigungspräparate, Lotionen, Öle, Puder, Sprays und Aerosole. Weitere Anwendungsformen sind z.B. Sticks, Shampoos und Duschbäder. Zusätzlich zu den Verbindungen der Formel I können der Formulierung beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer, Filmbildner, Verdickungsmittel, Feuchthaltemittel.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die kosmetischen Formulierungen können auch als emulgatorfreie, disperse Zubereitungen, vorliegen. Sie können beispielsweise Hydrodispersionen oder Pikkering-Emulsionen darstellen.

Die kosmetischen Formulierungen können auch als PIT-Emulsionen oder als Hydrogele vorliegen. Die kosmetischen Formulierungen können auch Liposomen, die beispielsweise Wirkstoffe umschliessen, enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Pasten, Salben, Gele und Cremes können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Seifen können die üblichen Trägerstoffe wie Alkalisalze von Fettsäuren, Salze von Fettsäurehalbestern, Fettsäureeiweißhydrolysaten, lsothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Weitere typisch kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in den kosmetischen Formulierungen verwendet werden können, sind entweder bekannt und käuflich erwerbbar oder können nach bekannten Verfahren synthetisiert werden.

Die erfindungsgemäße kosmetische Zubereitung eignet sich besonders zum Schutz menschlicher Haut vor den schädigenden Einflüssen der UV-Anteile im Sonnenlicht, daneben bieten sie auch Schutz gegen Alterungsprozesse der Haut sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer einer oder mehrerer Verbindungen der Formel I und gegebenenfalls noch weiteren Lichtschutzfiltern Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Formulierung kann auch zum Schutz der Haare gegen photochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die kosmetische Formulierung kann außer der oder den Verbindungen der Formel I und weiteren UV-Filtern verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte; organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend eine oder mehrere Verbindungen der Formel I aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Ferner wirken die Verbindungen der Formel I auch stabilisierend auf die Formulierung. Bei der Verwendung in entsprechenden Produkten bleiben diese daher auch länger stabil und verändern ihr Aussehen nicht. Insbesondere bleibt auch bei längerdauernder Anwendung bzw. längerer Lagerung die Wirksamkeit der Inhaltsstoffe, z.B. Vitamine, erhalten. Dies ist besonders vorteilhaft bei der Zusammensetzungen zum Schutz der Haut gegen die Einwirkung von UV-Strahlen, da diese Kosmetika besonders hohen Belastungen durch die UV-Strahlung ausgesetzt sind.

Ein weiterer Gegenstand der Erfindung betrifft die Stabilisierung von UV-Filtern. Eine bekannte und leistungsfähige Klasse von Lichtschutzfiltersubstanzen wird von den Dibenzoylmethanderivaten gebildet. Nachteilig ist jedoch, dass diese Substanzen sehr leicht durch UV-Licht zersetzt werden und damit ihre schützenden Eigenschaften verlorengehen. Als Beispiel für einen kommerziell erhältlichen Lichtschutzfilter aus dieser Verbindungsklasse sei das 4-(tert.-Butyl)-4'-methoxydibenzoylmethan angeführt, welches die unten gezeigte Struktur aufweist. 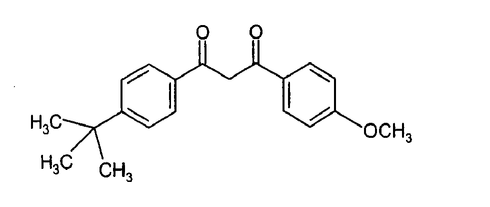 Überraschend hat sich nun gezeigt, dass Verbindungen der Formel I eine sehr gute Stabilisierungswirkung für die Dibenzoylmethane, insbesondere 4-(tert.-Butyl)-4-methoxydibenzoylmethan, aufweisen. Eine besonders hohe Stabilisierungswirkung wurde für Tilirosid gefunden. Hierbei kann Tilirosid als Reinsubstanz oder in Form eines Pflanzenextrakts zur Stabilisierung verwendet werden. In einer bevorzugten Ausführungsform wird Tilirosid in der Form eines Pflanzenextrakts, eines aufgereinigten Pflanzenextrakts oder in Form der aus dem Pflanzenextrakt hergestellten Reinsubstanz verwendet. Somit ist es nun möglich Lichtschutzmittel unter Verwendung von Dibenzoylmethanen herzustellen, die auch bei längerer Sonneneinwirkung, beispielsweise während eines mehrstündigen Sonnenbades, keine oder nur eine geringe Verringerung der Schutzwirkung gegen UV-Strahlen aufweisen.

Weiterhin Gegenstand der vorliegenden Erfindung sind Verbindungen der Formel I aus Anspruch 1 mit der Massgabe, dass an ein oder mehrere Hydroxygruppen der in den Substituenten Z₁ bis Z₁₀ genannten Reste jeweils unabhängig voneinander Sulfat oder Phosphat gebunden ist, wenn Z₅ ein Mono- oder Oligoglykosidrest ist, an den jeweils über eine Gruppe -O- ein oder mehrere Reste gebunden sind, die ausgewählt sind aus 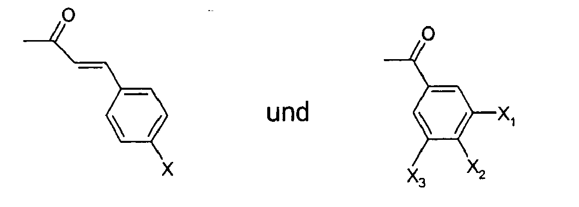 worin X, X₁, X₂ und X₃ jeweils unabhängig voneinander die in Anspruch 1 angegebenen Bedeutungen besitzen. Falls einer oder mehrere der Reste X, X₁, X₂ und X₃ OH bedeuten, können die genannten Sulfat- oder Phosphatgruppen auch an eine oder mehrere dieser Hydroxygruppen gebunden sein. Diese Verbindungen der Formel I werden im folgenden auch als Verbindungen der Formel I* bezeichnet.

In einer bevorzugten Ausführungsform ist Z₅ in den Verbindungen der Formel I* ein Mono- oder Oligoglykosidrest, an den jeweils über eine Gruppe -O- ein oder mehrere Reste 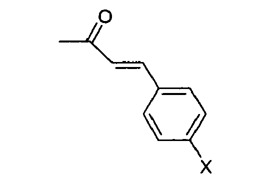 gebunden sind, worin X OH, CH₃COO, einen Alkoxyrest mit 1 bis 8 C-Atomen oder einen Monoglykosidrest bedeutet, und worin in den genannten Verbindungen an ein oder mehrere Hydroxygruppen der in den Substituenten Z₁ bis Z₁₀ genannten Reste jeweils unabhängig voneinander Sulfat gebunden ist. Falls X OH bedeutet, kann die genannte Sulfatgruppe auch an diese Hydroxygruppe gebunden sein.

Unter diesen Verbindungen ist insbesondere sulfatisiertes Tilirosid bevorzugt, also Tilirosid, das dadurch gekennzeichnet ist, dass an eine oder an mehrere Hydroxygruppen Sulfat gebunden ist.

Verbindungen der Formel I, insbesondere der Formel I*, eignen sich auch als Arzneimittel, z.B. in pharmazeutischen Formulierungen, wobei ihre obengenannten vorteilhaften Wirkungen, insbesondere als Radikalfänger und/oder Antioxidationsmittel, ausgenutzt werden. Sie wirken dabei z.B. unterstützend oder substituierend zu natürlichen Mechanismen, welche Radikale im Körper abfangen. Verbindungen der Formel I, insbesondere der Formel I*, können in bestimmten Fällen z.B. auch zur Verhütung bestimmter Krebsarten angewendet werden.

Gegenstand der Erfindung ist ferner die Verwendung der Verbindungen der Formel I, insbesondere der Formel I*, und/oder ihrer physiologisch unbedenklichen Salze zur Herstellung pharmazeutischer Zubereitungen, insbesondere auf nicht-chemischem Wege. Hierbei können sie zusammen mit mindestens einem festen, flüssigen und/oder halbflüssigen Träger-oder Hilfsstoff und gegebenenfalls in Kombination mit einem oder mehreren weiteren Wirkstoffen in eine geeignete Dosierungsform gebracht werden.

Gegenstand der Erfindung sind ferner pharmazeutische Zubereitungen, enthaltend mindestens eine Verbindung der Formel I, insbesondere der Formel I*, und/oder eines ihrer physiologisch unbedenklichen Salze.

Diese Zubereitungen können als Arzneimittel in der Human- oder Veterinärmedizin verwendet werden. Als Trägerstoffe kommen organische oder anorganische Substanzen in Frage, die sich für die enterale (z.B. orale), parenterale oder topische Applikation eignen und mit den Verbindungen der Formel I, insbesondere der Formel I*, nicht reagieren, beispielsweise Wasser, pflanzliche Öle, Benzylalkohole, Alkylenglykole, Polyethylenglykole, Glycerintriacetat, Gelatine, Kohlehydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die Verbindungen der Formel I, insbesondere der Formel I*, können auch lyophilisiert und die erhaltenen Lyophilisate z.B. zur Herstellung von Injektionspräparaten verwendet werden. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks- und/oder mehrere weitere Wirkstoffe enthalten, z.B. ein oder mehrere Vitamine.

Dabei werden die Verbindungen der Formel I, insbesondere der Formel I*, in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Die pharmazeutischen Formulierungen enthaltend eine oder mehrere Verbindungen der Formel I, insbesondere der Formel I*, können mit Hilfe von Techniken hergestellt werden, die dem Fachmann wohl bekannt sind.

Die vorteilhaften Eigenschaften der Verbindungen der Formel I, insbesondere der Formel I*, können z.B. auch bei ihrer Verwendung in Nahrungsmitteln oder als Nahrungsergänzungsmittel oder als "functional food" ausgenutzt werden. Beispielsweise können die Verbindungen der Formel I, insbesondere der Formel I*, die weiterhin in dem Nahrungsmittel, dem Nahrungsergänzungsmittel oder dem "functional food" enthaltenen Verbindungen oder auch den Organismus vor Oxidation bzw. vor der Einwirkung von Radikalen schützen.

Ein weiterer Gegenstand der Erfindung betrifft Nahrungsmittel, die mit einer oder mehreren Verbindungen der Formel I, insbesondere der Formel I*, angereichert worden sind sowie Nahrungsergänzungsmittel, die eine oder mehrere Verbindungen der Formel I, insbesondere der Formel I*, enthalten.

Die weiteren zu Nahrungsmitteln ausgeführten Erläuterungen gelten sinngemäß auch für Nahrungsergänzungsmittel und für "functional food". Die Nahrungsmittel, die nach der vorliegenden Erfindung mit einer oder mehreren Verbindungen der Formel I, insbesondere der Formel I*, angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren. Nahrungsmittel, die nach der vorliegenden Erfindung mit einer oder mehreren Verbindungen der Formel I, insbesondere der Formel I*, angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensauce, Tomatenpüree, usw.. Weitere Beispiele für Nahrungsmittel, die nach der vorliegenden Erfindung mit einer oder mehreren Verbindungen der Formel I, insbesondere der Formel I*, angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I, insbesondere der Formel I*, angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I, insbesondere der Formel I*, angereichert werden können, seien Nahrungsmittelzubereitungen, beispielsweise zubereitete Zerealien, Gebäck, Mischgetränke, speziell für Kinder zubereitete Nahrungsmittel wie Joghurt, Diätnahrungsmittel, kalorienarme Nahrungsmittel oder Tierfutter genannt.

Die Nahrungsmittel, die nach der vorliegenden Erfindung mit einer oder mehreren Verbindungen der Formel I, insbesondere der Formel I*, angereichert werden können, umfassen somit alle genießbaren Kombinationen von Kohlehydraten, Lipiden, Proteinen, anorganischen Elementen, Spurenelementen, Vitaminen, Wasser und aktiven Metaboliten von Pflanzen und Tieren.

Die Nahrungsmittel, die nach der vorliegenden Erfindung mit einer oder mehreren Verbindungen der Formel I, insbesondere der Formel I*, angereichert werden können sowie die Nahrungsergänzungsmittel, die eine oder mehrere Verbindungen der Formel I, insbesondere der Formel I*, enthalten, werden vorzugsweise oral angewendet, z.B. in Form von Essen, Pillen, Tabletten, Kapseln, Pulver, Syrups, Lösungen oder Suspensionen.

Die mit einer oder mehreren Verbindungen der Formel I, insbesondere der Formel I*, angereicherten Nahrungsmittel können mit Hilfe von Techniken hergestellt werden, die dem Fachmann wohl bekannt sind.

Auch ohne weitere Ausführungen wird davon ausgegangen, dass ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die vorliegende Erfindung verdeutlichen. Sie sind jedoch keinesfalls als limitierend zu betrachten.

Alle Verbindungen oder Komponenten, die in den kosmetischen Formulierungen verwendet werden können, sind entweder bekannt und käuflich erhältlich oder können nach bekannten Methoden synthetisiert werden.

Die INCl-Namen der verwendeten Rohstoffe sind wie folgt (die INCl-Namen werden definitionsgemäß in Englischer Sprache angegeben):

| **Rohstoff** | **INCI-Name** |
|---|---|
| Abil WE 09 | Polyglyceryl-4-Isostearate, Cetyl Dimethicone Copolyol, Hexyl Laurate |
| Antaron V-220 | PVP/Eicosene Copolymer |
| Arlacel 80 | Sorbitan Oleate |
| Arlacel 165 V | Glyceryl Stearate, PEG-100 Stearate |
| Avocadoöl | Persea Gratissima |
| Bienenwachs | Beeswax |
| Biobase^{™} EP | Glyceryl Stearate, Cetearyl Alcohol, Sodium Stearoyl Lactylate, Lecithin |
| Carbopol ETD 2050 | Carbomer |
| Cetiol V | Decyl Oleate |
| Cetylalkohol | Cetyl Alcohol |
| Cetylisononanoat | Cetyl Isononanoate |
| Cutina HR | Hydrogenated Castor Oil |
| Dimeticon | Dimethicone |
| Eusolex^{®}232 | Phenylbenzimidazole Sulfonic Acid |
| Eusolex^{®} 2292 | Octyl Methoxycinnamate, BHT |
| Eusolex^{®} 6300 | 4-Methylbenzylidene Camphor |
| Eusolex 8300 | 4-Methylbenzylidene |
| Eusolex^{®} 9020 | Butyl Methoxydibenzoylmethane |
| Eusolex^{®}HMS | Homosalate |
| Eusolex T-Aqua | Aqua (Water), Titanium Dioxide, Alumina, Sodium Metaphosphate, Phenoxyethanol, Sodium Methyl-paraben |
| Eutanol G | Octyldodecanol |
| Germaben II | Propylene Glycol, Diazolidinyl Urea, Methylparaben, Propylparaben |
| Germaben II-E | Propylene Glycol, Diazolidinyl Urea, Methylparaben, Propylparaben |
| Glycerin | Glycerin |
| Glycerin (87%) | Glycerin |
| Glycerin (87% reinst) | Glycerin |
| Glycerin, wasserfrei | Glycerin |
| Hetester PHA | Propylene Glycol Isoceteth-3 Acetate |
| Hexyllaurat | Hexyl Laurate |
| Imwitor 960 K Schuppen | Glyceryl Stearate SE |
| Isolan PDI | Diisostearoyl Polyglyceryl-3-Diisostearat |
| Isopropylmyristat | Isopropyl Myristate |
| Isopropylpalmitat | Isopropyl Palmitate |
| Jojobaöl | Buxus Chinensis (Jojoba Oil) |
| Karion F flüssig | Sorbitol |
| Keltrol RD | Xanthan Gum |
| Magnesiumsulfat | Magnesium Sulfate |
| Magnesiumsulfat-Heptahydrat | Magnesium Sulfate |
| Methyl-4-hydroxybenzoat | Methylparaben |
| Miglyol 812 | Caprylic/Capric Triglyceride |
| Miglyol 812 N | Caprylic/Capric Triglyceride |
| Miglyol 812, Neutralöl | Caprylic/Capric Triglyceride |
| Mirasil CM5 | Cyclomethicone |
| Mirasil DM 350 | Dimethicone |
| Montanov 68 | Cetearyl Alcohol, Cetearyl Glucoside |
| Natriumchlorid | Sodium Chloride |
| Natronlauge, 10%ig | Sodium Hydroxide |
| Oxynex^{®}K | PEG-8, Tocopherol, Ascorbyl Palmitate, Ascorbic Acid, Citric Acid |
| Panthenol-D | Panthenol |
| Paracera M | Microwax |
| Paraffinöl, fl. | Mineral Oil |
| Parfümöl TND-2417 | Parfum |
| Pemulen TR-1 | Acrylates/C₁₀₋₃₀ Alkyl Acrylate Crosspolymer |
| Pemulen^{®} TR-2 | Acrylates/C₁₀₋₃₀ Alkyl Acrylate Crosspolymer |
| Performa^{®} V 825 | Synthetic Wax |
| Polyglyceryl-2-Dipolyhydroxy- | Polyglyceryl-2 Dipolyhydroxystearate |
| stearat | |
| Prisorine 2021 | Isopropyl Isostearate |
| Propandiol-1,2 | Propylene Glycol |
| Propyl-4-hydroxybenzoat | Propylparaben |
| Rhodicare S | Xanthan Gum |
| RonaCare^{™} ASC III | Aqua, Lecithin, Dipalmitoyl Hydroxyproline, Phenoxyethanol, Tall Oil Sterol, Linoleic Acid, Tocopherol, Sodium Ascorbate, Mannitol, Methylparaben, Ethylparaben, Propylparaben, Butylparaben |
| RonaCare^{™} Bisabolol | Bisabolol |
| RonaCare^{™} Ectoin | Ectoin |
| RonaCare^{™} LPO | Lauryl p-Cresol Ketoxime |
| RonaCare^{™} Tocopherolacetat | Tocopheryl Acetate |
| Sepigel 305 | Polyacrylamide, C₁₃₋₁₄ lsoparaffin, |
| SFE 839 | Cyclopentasiloxane, Dimethicone/ Vinyldimethicone Crosspolymer |
| Shea Butter | Shea Butter |
| Steareth-2 | Steareth-2 |
| Steareth-10 | Steareth-10 |
| Stearinsäure | Stearic Acid |
| DL-α-Tocopherolacetat | Tocopherol Acetate |
| Triethanolamin | Triethanolamine |
| Triethanolamin reinst | Triethanolamine |
| Wasser, demineralisiert | Aqua (Water) |
| Zinkstearat | Zinc Stearate |

### Beispiele

### Beispiel A

### Herstellung von sulfatisiertem Tilirosid, Natriumsalz

29,7 g Tilirosid (50 mmol) werden unter Rühren mit 200 ml Wasser und 19,4 g 32%iger Natronlauge (155,2 mmol) versetzt. Anschließend wird 19,9 g Pyridinsulfon (125 mmol) zugegeben und der pH-Wert durch Zugabe von 32%iger Natronlauge auf pH-Wert 8 eingestellt. Der Reaktionsansatz wird 12 h unter N₂ gerührt, danach filtriert und das Filtrat unter vermindertem Druck auf 50 g eingeengt (T = 60 °C; p = 100 mbar). Zu dem eingeengten Filtrat werden 250 ml Methanol innerhalb von 1 h zugetropft und der ausgefallene Feststoff (Natriumsulfat) abfiltriert. Nach dem Trocknen wird sulfatisiertes Tilirosid, Natriumsalz erhalten.

### Beispiel 1

### Lotion (W/O) zum Auftragen auf die Haut

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 0,5 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird
Phase B langsam zu Phase A gegeben. Nach dem Homogenisieren wird unter Rühren abgekühlt. Bei einer Temperatur von 40°C werden Parfümstoffe zugegeben.

### Als Konservierungsmittel werden verwendet:

0,05 % Propyl-4-hydroxybenzoat
0,15 % Methyl-4-hydroxybenzoat

### Beispiel 2

### Lotion (W/O) zum Auftragen auf die Haut

| | | **Gew.%** |
|---|---|---|
| **A** | Polyglyceryl-2-dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | | |
| **B** | sulfatisiertes Tilirosid, Natriumsalz (Beispiel A) | 1,0 |
| | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird
Phase B langsam zu Phase A gegeben. Nach dem Homogenisieren wird unter Rühren abgekühlt. Bei einer Temperatur von 40°C werden Parfümstoffe zugegeben.

### Als Konservierungsmittel werden verwendet:

0,05 % Propyl-4-hydroxybenzoat
0,15 % Methyl-4-hydroxybenzoat

### Beispiel 3

### Sonnenschutzspray (O/W)

| | | | **Gew.%** |
|---|---|---|---|
| **A** | Eusolex 9020 | (1) | 2,0 |
| | Eusolex^{®}HMS | (1) | 7,0 |
| | Steareth-2 | | 0,4 |
| | Steareth-10 | | 0,8 |
| | Pemulen^{®} TR-2 | (2) | 0,18 |
| | Hetester PHA | (3) | 5,0 |
| | Performa^{®} V 825 | (4) | 0,8 |
| | Dimeticon | | 1,0 |
| | Oxynex^{®}K | (1) | 0,1 |
| | | | |
| **B** | sulfatisiertes Tilirosid, Natriumsalz (Beispiel A) | | 1,0 |
| | Eusolex^{®}232 | (1) | 1,0 |
| | Triethanolamin | (1) | 0,9 |
| | Propandiol-1,2 | (1) | 2,0 |
| | Wasser, demineralisiert | | ad 100 |

### Herstellung

Phase A
   Die Bestandteile der Phase A mit Ausnahme von Permulen^{®}TR-2 werden zusammengegeben und auf 80°C erwärmt. Anschließend wird das Permulen^{®}TR-2 unter Rühren zugegeben.
Phase B
   Das Wasser wird mit dem Triethanolamin vermischt und anschließend Eusolex^{®}232 unter Rühren zugegeben. Sobald sich alles gelöst hat, werden die weiteren Bestandteile der Phase B zugegeben und anschließend die Mischung auf 80°C erwärmt.

### Herstellung des Sonnenschutzmittels

Zur Phase A wird unter Rühren langsam die Phase B gegeben. Nach dem Homogenisieren wird unter Rühren abgekühlt.

### Als Konservierungsmittel werden verwendet:

0,05 % Propyl-4-hydroxybenzoat
0,15 % Methyl-4-hydroxybenzoat

### Bezugsquellen

(1) Merck KGaA
(2) BF Goodrich
(3) Bernel
(4) New Phase

### Beispiel 4

### Sonnenschutzcreme (O/W)

| | | | **Gew.%** |
|---|---|---|---|
| **A** | Eusolex^{®} 2292 | (1) | 4,0 |
| | Eusolex^{®} 9020 | (1) | 1,0 |
| | Eusolex^{®} 6300 | (1) | 1,0 |
| | Stearinsäure | (1) | 2,5 |
| | Imwitor 960 K Schuppen | (2) | 1,0 |
| | Antaron V-220 | (3) | 2,0 |
| | Cetylalkohol | (1) | 0,3 |
| | Miglyol 812 N | (4) | 5,5 |
| | Jojobaöl | (5) | 2,0 |
| | Pemulen TR-1 | (6) | 0,25 |
| | Tilirosid | (1) | 1,0 |
| | | | |
| **B** | Glycerin, wasserfrei | (1) | 3,0 |
| | Propyl-4-hydroxybenzoat | (1) | 0,1 |
| | Methyl-4-hydroxybenzoat | (1) | 0,2 |
| | Keltrol RD | | 0,4 |
| | Wasser, demineralisiert | | ad 100 |
| | | | |
| **C** | Triethanolamin reinst | (1) | 0,9 |
| | | | |
| **D** | SFE 839 | (7) | 5,0 |
| | Arlace 80 | (8) | 0,3 |

### Herstellung

Phase A wird gemischt und auf 75°C erhitzt. Phase B wird gemischt und auf 70°C erhitzt. Anschliessend wird Phase A zu Phase B gegeben, das Gemisch homogenisiert und unter Rühren auf 45 °C abgekühlt. Danach werden Phase C und Phase D unter Rühren zugegeben.

### Bezugsquellen

(1) Merck KGaA
(2) Condea Chemie GmbH
(3) ISP Global Technologies
(4) Condea Chemie GmbH
(5) Gustav Heess GmbH
(6) BF Goodrich GmbH
(7) GE Silicones Holland
(8) Uniqema

### Beispiel 5

### Lotion (W/O) zum Auftragen auf die Haut

| | | **Gew.%** |
|---|---|---|
| **A** | 4,6,3',4'-Tetrahydroxybenzylcoumaranon-3 | 1,0 |
| | Polyglyceryl-2-Dipolyhydroxystearat | 5,0 |
| | Bienenwachs | 0,5 |
| | Zinkstearat | 0,5 |
| | Hexyllaurat | 9,0 |
| | Cetylisononanoat | 6,0 |
| | Shea Butter | 0,5 |
| | DL-α-Tocopherolacetat | 1,0 |
| | Tilirosid | 1,0 |
| | | |
| **B** | Glycerin | 5,0 |
| | Magnesiumsulfat-Heptahydrat | 1,0 |
| | Konservierungsmittel | q.s. |
| | Wasser, demineralisiert | ad 100 |

### Herstellung

Phase A wird auf 75°C und Phase B auf 80°C erwärmt. Unter Rühren wird
Phase B langsam zu Phase A gegeben. Nach dem Homogenisieren wird unter Rühren abgekühlt. Bei einer Temperatur von 40°C werden Parfümstoffe zugegeben.

### Als Konservierungsmittel werden verwendet:

0,05 % Propyl-4-hydroxybenzoat
0,15 % Methyl-4-hydroxybenzoat

### Beispiel 6

### O/W After Sun Lotion

| | | | **Gew.%** |
|---|---|---|---|
| **A** | RonaCare^{™} Bisabolol | (1) | 0,3 |
| | Montanov 68 | (2) | 4,0 |
| | Miglyol 812, Neutralöl | (3) | 12,0 |
| | Mirasil CM5 | (4) | 2,0 |
| | Mirasil DM 350 | (4) | 1,0 |
| | Tilirosid | | 1,0 |
| | | | |
| **B** | Wasser, demineralisiert | | ad 100 |
| | Glycerin (87% reinst) | (1) | 3,0 |
| | Konservierungsmittel | | q.s. |
| | RonaCare^{™} Ectoin | (1) | 1,0 |
| | | | |
| **C** | Rhodicare S | (4) | 0,5 |

### Herstellung

Phasen A und B werden getrennt auf 75°C erhitzt. Phase C wird bei 75°C unter Rühren langsam zu Phase B zugegeben und es wird gerührt, bis eine homogene Mischung entsteht. Anschließend wird Phase A zu der Mischung B/C gegeben und homogenisiert. Unter Rühren wird die erhaltene Mischung auf Raumtemperatur abgekühlt.

### Als Konservierungsmittel werden verwendet:

0,05 % Propyl-4-hydroxybenzoat
0,15 % Methyl-4-hydroxybenzoat

### Bezugsquellen

(1) Merck KGaA
(2) Seppic
(3) Condea Chemie GmbH
(4) Rhodia GmbH

### Beispiel 7

### Sonnenschutzlotion (W/O)

| | | | |
|---|---|---|---|
| | | | **Gew.%** |
| **A** | Eusolex 8300 | (1) | 4,0 |
| | Eusolex 2292 | (1) | 7,0 |
| | Abil WE 09 | (2) | 5,0 |
| | Jojobaöl | (3) | 3,0 |
| | Cetiol V | (4) | 3,0 |
| | Prisorine 2021 | (5) | 2,0 |
| | Paracera M | | 1,0 |
| | Miglyol 812, Neutralöl | (6) | 3,0 |
| | Propyl-4-hydroxybenzoat | (1) | 0,05 |
| | Tilirosid | | 1,0 |
| | | | |
| **B** | Eusolex T-Aqua | (1) | 16,0 |
| | Glycerin (87% reinst) | (1) | 2,0 |
| | Natriumchlorid | (1) | 0,4 |
| | RonaCare^{™} Ectoin | (1) | 1,0 |
| | Wasser, demineralisiert | | ad 100 |
| | Methyl-4-hydroxybenzoat | (1) | 0,15 |

### Herstellung

Phase B wird auf 80°C und Phase A wird auf 75°C erhitzt. Phase B wird langsam in Phase A eingerührt. Das Gemisch wird homogenisiert und unter Rühren abgekühlt.

### Bezugsquellen

(1) Merck KGaA
(2) Goldschmidt AG
(3) Gustav Heess GmbH
(4) Cognis GmbH
(5) Uniqema
(6) Condea Chemie GmbH

### Beispiel 8

Aus folgenden Komponenten wird eine Creme (O/W), enthaltend Ectoin, hergestellt:

| | | | **Gew.%** |
|---|---|---|---|
| **A** | Paraffin, dünnflüssig | (1) | 8,0 |
| | Isopropylmyristat | (1) | 4,0 |
| | Mirasil CM5 | (2) | 3,0 |
| | Stearinsäure | (1) | 3,0 |
| | Arlacel 165 V | (3) | 5,0 |
| | Tilirosid | | 1,0 |
| | | | |
| **B** | Glycerin (87%) | (1) | 3,0 |
| | Germaben II | (4) | 0,5 |
| | Wasser, demineralisiert | | ad 100 |
| | | | |
| **C** | RonaCare^{™} Ectoin | (1 ) | 1,0 |

### Herstellung

Zunächst werden die Phasen A und B getrennt auf 75°C erwärmt. Danach wird Phase A unter Rühren langsam zu Phase B gegeben und solange gerührt, bis eine homogene Mischung entsteht. Nach Homogenisierung der Emulsion wird unter Rühren auf 30°C abgekühlt. Anschließend wird auf 35°C erwärmt, die Phase C zugegeben und bis zur Homogenität gerührt.

### Bezugsquellen

(1) Merck KGaA
(2) Rhodia
(3) Uniqema
(4) ISP

### Beispiel 9

### Topische Zusammensetzung als W/O-Emulsion

| | | | **Gew.%** |
|---|---|---|---|
| **A** | Isolan PDI | (2) | 3,0 |
| | Paraffinöl, fl. | (1) | 17,0 |
| | Isopropylmyristat | | 5,0 |
| | Bienenwachs | | 0,2 |
| | Cutina HR | (2) | 0,3 |
| | Tilirosid | | 1,0 |
| | | | |
| **B** | Wasser, demineralisiert | | ad 100 |
| | Glycerin (87%) | | 4,0 |
| | Magnesiumsulfat | | 1,0 |
| | Germaben II-E | (3) | 1,0 |
| | | | |
| **C** | RonaCare^{™} LPO | (1) | 2,0 |

### Herstellung

Die Phasen A und B werden auf 75°C erwärmt. Phase B wird unter Rühren zu Phase A gegeben. Anschließend wird das Gemisch bei 9000upm 2 Min. mit dem Turrax homogenisiert. Das erhaltene Gemisch wird auf 30 bis 35°C abgekühlt, und C wird eingerührt.

### Bezugsquellen

(1) Merck KGaA
(2) Goldschmidt AG
(3) ISP

### Beispiel 10

### After-Sun-Lotion (O/W)

| | | | **Gew.%** |
|---|---|---|---|
| **A** | Biobase^{™} EP | (4) | 4,5 |
| | Isopropylpalmitat | (1) | 3,0 |
| | Cetiol V | (1) | 2,5 |
| | Miglyol 812 | (2) | 9,0 |
| | Carbopol ETD 2050 | (3) | 0,3 |
| | RonaCare^{™} LPO | (2) | 1,0 |
| | Tilirosid | | 1,0 |
| | | | |
| **B** | Wasser, demineralisiert | | ad 100 |
| | Glycerin (87% reinst) | (2) | 3,0 |
| | Konservierungsmittel | | q.s. |
| | | | |
| **C** | Natronlauge, 10%ig | (2) | |

### Herstellung

Die Phasen A und B werden getrennt auf 70°C erwärmt. Anschliessend wird Phase B unter Rühren zu Phase A gegeben. Danach wird das Gemisch homogenisiert, mit Natronlauge neutralisiert und unter Rühren abgekühlt.

### Als Konservierungsmittel werden verwendet:

0,05 % Propyl-4-hydroxybenzoat
0,15 % Methyl-4-hydroxybenzoat

### Bezugsquellen

(1) Cognis GmbH
(2) Merck KGaA
(3) BF Goodrich GmbH
(4) Tri-K Industries, Inc.

### Beispiel 11

### Hautpflegegel (O/W)

| | | | **Gew.%** |
|---|---|---|---|
| **A** | Eusolex^{®} 6300 | (1) | 1,0 |
| | RonaCare^{™} Tocopherolacetat | (1) | 1,0 |
| | Avocadoöl | (2) | 5,0 |
| | Jojobaöl | (2) | 5,0 |
| | Miglyol 812 N | (3) | 3,0 |
| | Eutanol G | (4) | 5,0 |
| | Sepigel 305 | (5) | 3,0 |
| | Tilirosid | (1) | 0,5 |
| | | | |
| **B** | Wasser, demineralisiert | | ad 100 |
| | Karion F flüssig | (1) | 5,0 |
| | Panthenol-D | (6) | 1,0 |
| | | | |
| **C** | RonaCare^{™} ASC III | (1) | 4,0 |
| | Parfümöl TND-2417 | (7) | 0,1 |
| | Konservierungsmittel | | q.s. |

### Herstellung

Die Phasen A und B werden separat vorgelöst. Anschliessend wird Phase B unter Rühren zu Phase A gegeben und nach und nach die Bestandteile der Phase C zugegefügt.

### Als Konservierungsmittel werden verwendet:

0,05 % Propyl-4-hydroxybenzoat
0,15 % Methyl-4-hydroxybenzoat

### Bezugsquellen

(1) Merck KGaA
(2) Gustav Heess GmbH
(3) Condea Chemie GmbH
(4) Cognis GmbH
(5) Interogana GmbH
(6) Hoffmann-La Roche AG
(7) Takasago

## Patentansprüche

1. Nahrungsmittel, angereichert mit einer oder mehrere Verbindungen der Formel lA 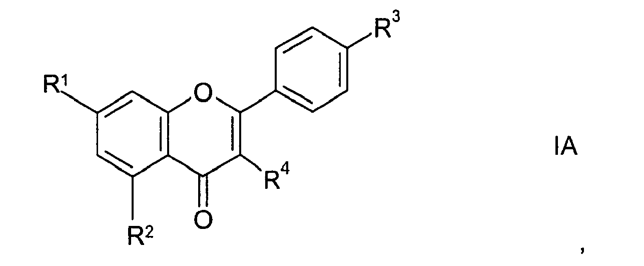 worin
R¹, R²
und R³ jeweils unabhängig voneinander OH, CH₃COO, einen Alkoxyrest mit 1 bis 8 C-Atomen oder einen Monoglykosidrest bedeuten,
R⁴ ein Mono- oder Diglykosidrest ist, wobei an den Glykosidrest jeweils über eine Gruppe -O- mindestens eine Gruppe ausgewählt aus 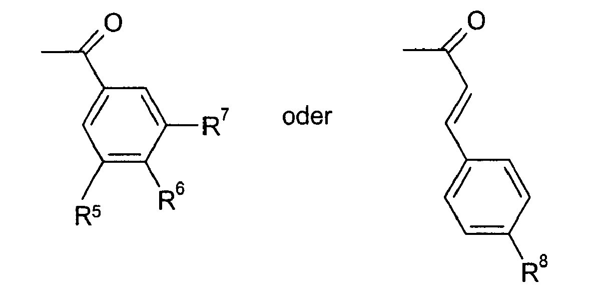 gebunden ist,
R⁵ R⁶,
R⁷und R⁸ jeweils unabhängig voneinander die Bedeutung der Reste R¹ bis R³ besitzen, und
worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann, wobei die eine oder mehreren Verbidungen der Formel IA in Form einer synthetisch gewonnenen Substanz oder in Form der aus einem Pflanzenextrakt gewonnenen Reinsubstanz vorliegen.

2. Nahrungsergänzungsmittel enthaltend eine oder mehrere der Verbindungen der Formel IA 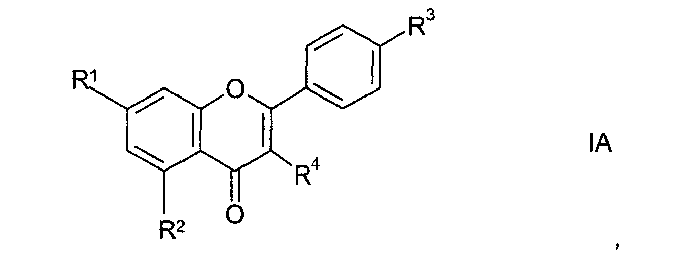 worin
R¹ R²
und R³ jeweils unabhängig voneinander OH, CH₃COO, einen Alkoxyrest mit 1 bis 8 C-Atomen oder einen Monoglykosidrest bedeuten,
R⁴ein Mono- oder Diglykosidrest ist, wobei an den Glykosidrest jeweils über eine Gruppe -O- mindestens eine Gruppe ausgewählt aus 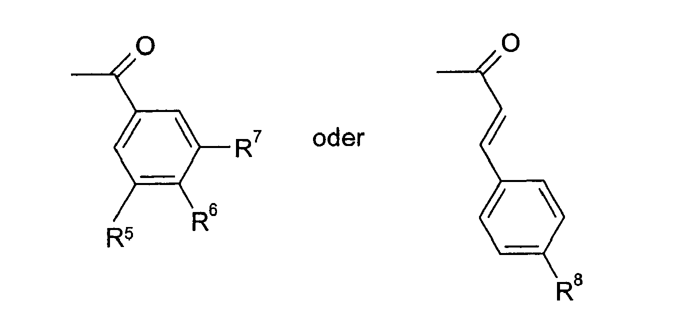 gebunden ist,
R⁵, R⁶,
R⁷ und R⁸ jeweils unabhängig voneinander die Bedeutung der Reste R¹ bis R³ besitzen, und
worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann, wobei die eine oder mehreren Verbidungen der Formel IA in Form einer synthetisch gewonnenen Substanz oder in Form der aus einem Pflanzenextrakt gewonnenen Reinsubstanz vorliegen.

3. Nahrungsmittel oder Nahrungsergänzungsmittel nach Anspruch 1 oder 2, **dadurch gekennzeichnet, dass** die Verbindungen der Formel IA ausgewählt sind aus den Verbindungen der Formeln IA1 bis IA13

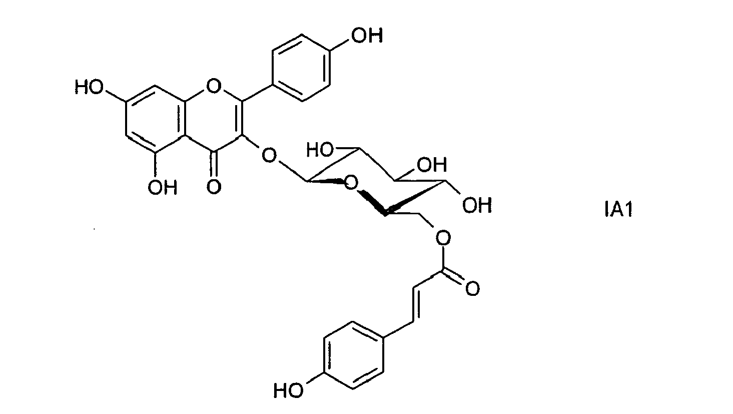

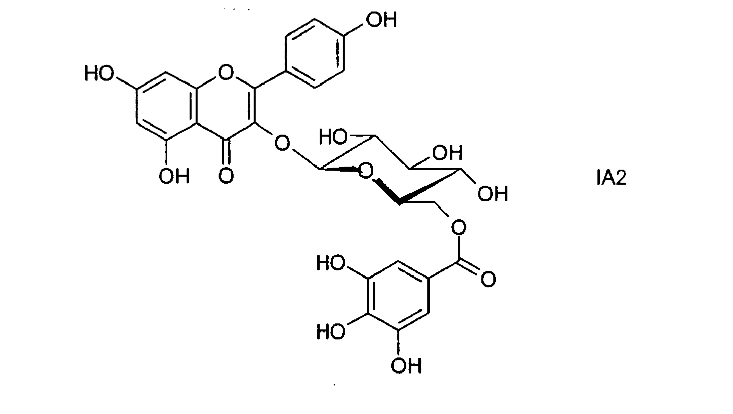

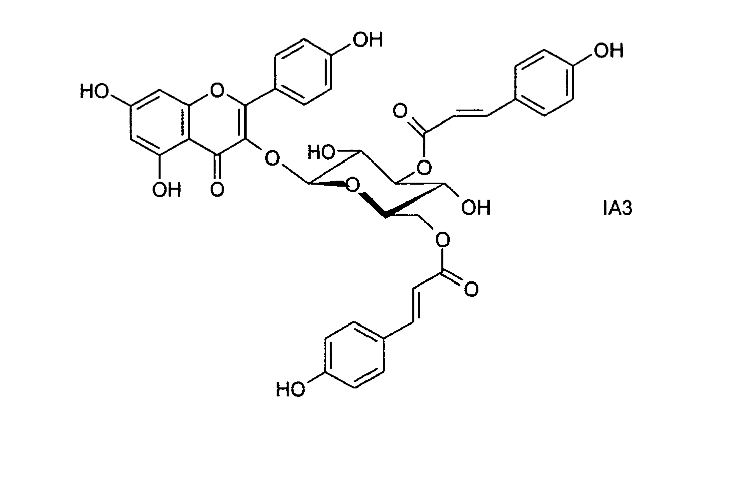

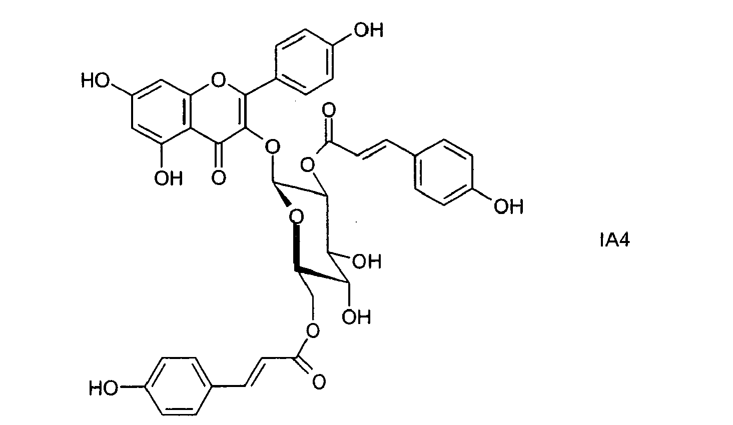

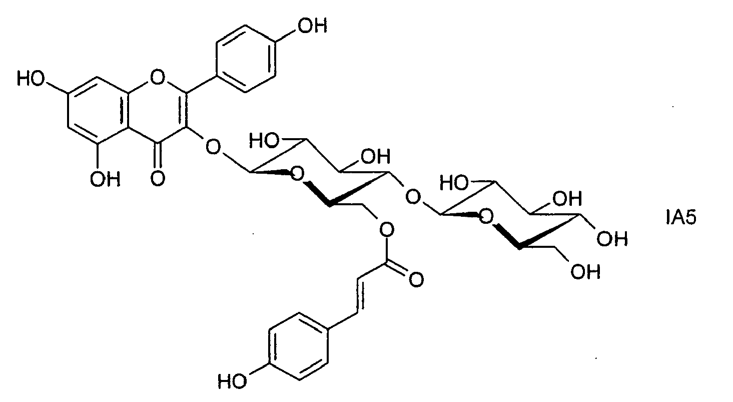

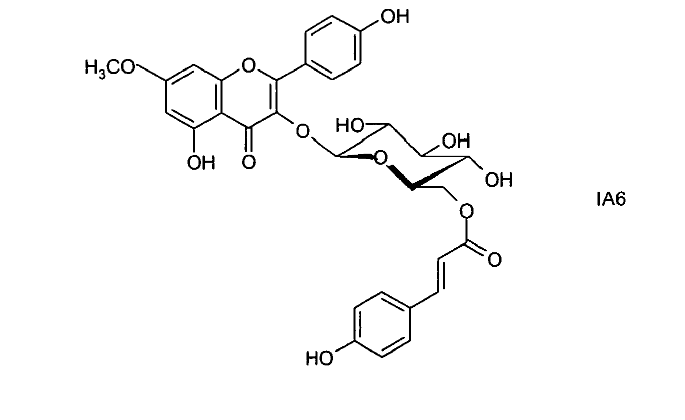

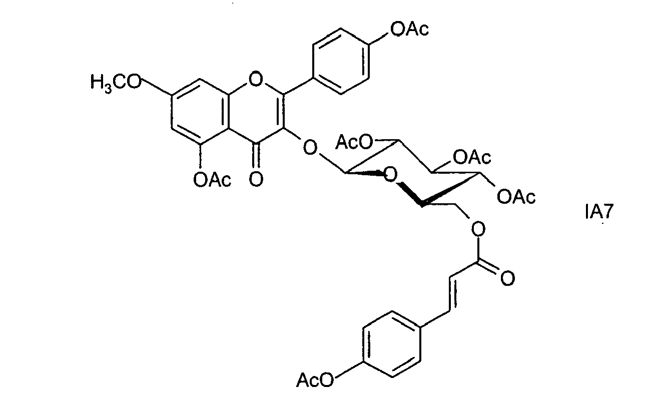

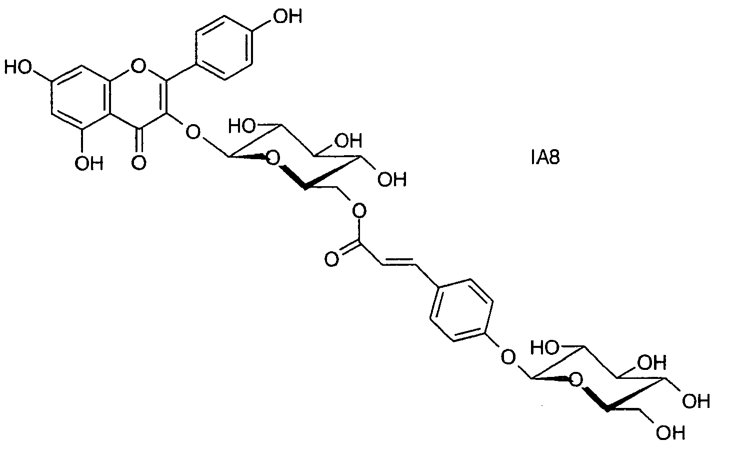

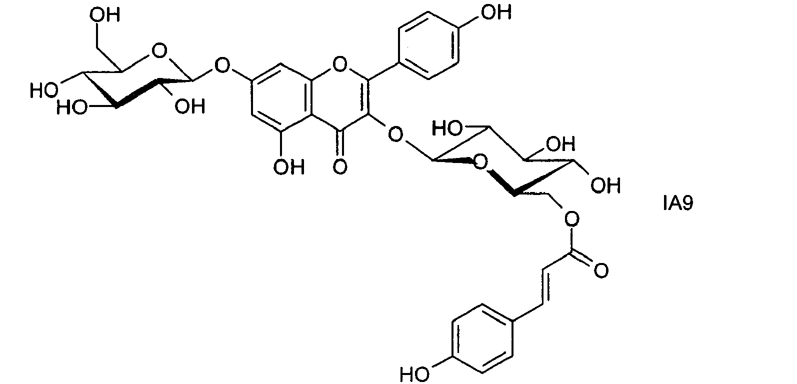

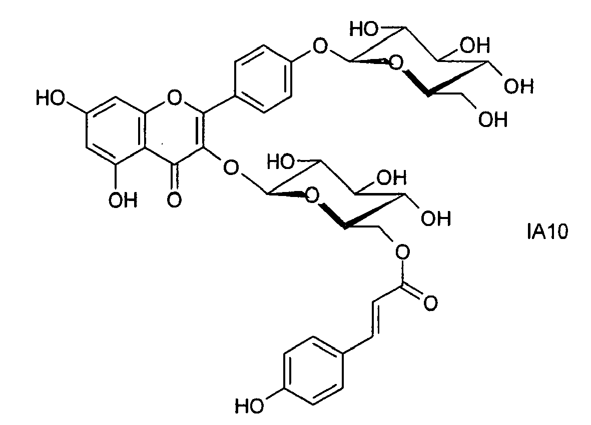

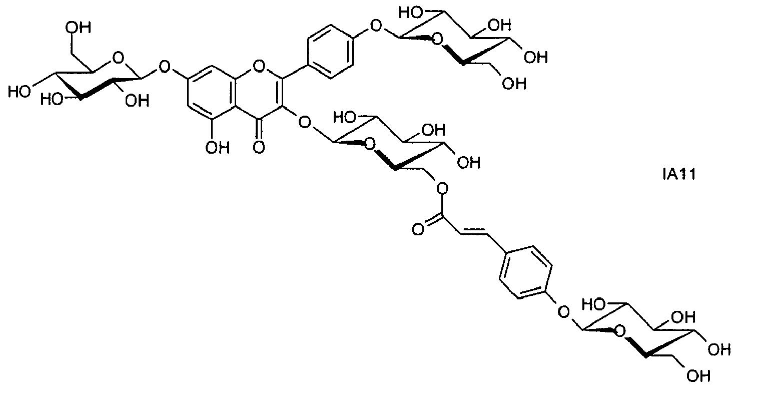

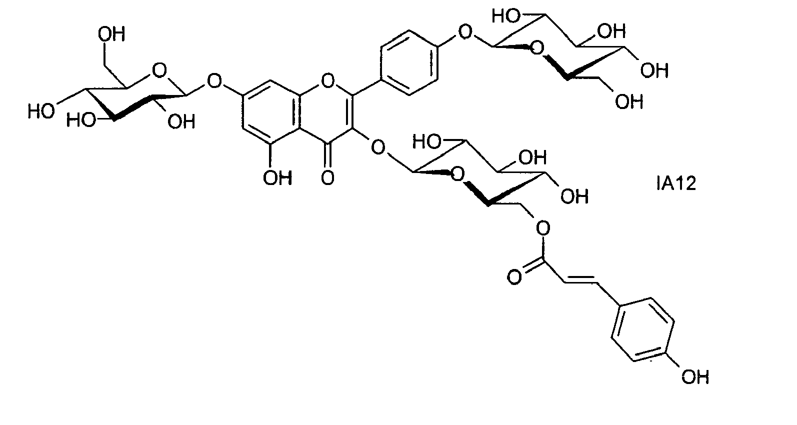

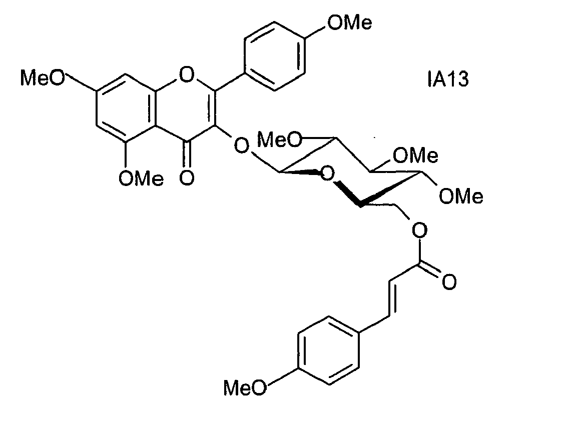

4. Nahrungsmittel oder Nahrungsergänzungsmittel nach Anspruch 1 oder 2, **dadurch gekennzeichnet, dass** die Verbindung der Formel IA Tilirosid ist.

5. Nahrungsmittel oder Nahrungsergänzungsmittel nach Anspruch 1 oder 2, **dadurch gekennzeichnet, dass** die Verbindung der Formel lA Tilirosid ist, das **dadurch gekennzeichnet ist, dass** an eine oder mehrere Hydroxygruppen Sulfat gebunden ist.

6. Verwendung von einer oder mehreren Verbindungen der Formel IA 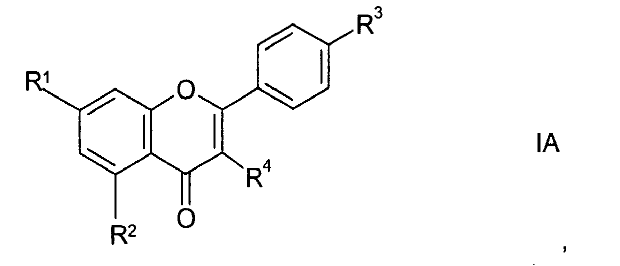 wobei die eine oder mehreren Verbidungen der Formel IA in Form einer synthetisch gewonnenen Substanz oder in Form der aus einem Pflanzenextrakt gewonnenen Reinsubstanz vorliegen und worin
R¹, R²
und R³ jeweils unabhängig voneinander OH, CH₃COO, einen Alkoxyrest mit 1 bis 8 C-Atomen oder einen Monoglykosidrest bedeuten,
R⁴ein Mono- oder Diglykosidrest ist, wobei an den Glykosidrest jeweils über eine Gruppe -O- mindestens eine Gruppe ausgewählt aus 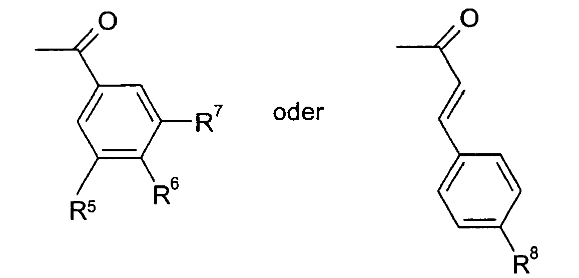 gebunden ist,
R⁵, R⁶ ,
R⁷ und R⁸ jeweils unabhängig voneinander die Bedeutung der Reste R¹ bis R³ besitzen, und
worin ein oder mehrere Wasserstoffatome in den OH-Gruppen des oder der Glykosidreste jeweils unabhängig voneinander auch durch Acetyl oder durch Alkylreste mit 1 bis 8 C-Atomen ersetzt sein können und wobei an ein oder mehrere Hydroxygruppen der Verbindungen der Formel IA jeweils unabhängig voneinander auch Sulfat oder Phosphat gebunden sein kann,
in Nahrungsmitteln oder als Nahrungsergänzungsmittel.

7. Verwendung nach Anspruch 6, **dadurch gekennzeichnet, dass** Tilirosid verwendet wird.

8. Verwendung nach Anspruch 6 oder 7, **dadurch gekennzeichnet, dass** Tilirosid verwendet wird, das **dadurch gekennzeichnet ist, dass** an eine oder mehrere Hydroxygruppen Sulfat gebunden ist.
